# EUROPÄISCHE PATENTSCHRIFT

(11) **EP 0 973 908 B1**
(45) Veröffentlichungstag und Bekanntmachung des Hinweises auf die Patenterteilung: **01.09.2004**
(21) Anmeldenummer: 98931930.6
(22) Anmeldetag: 15.04.1998
(51) Int. Cl.: C12N 15/29, C12N 15/82, C07K 14/415, C12N 5/10, A01H 5/00, A01H 5/10

(54) **PFLANZEN MIT GESTEUERTER SEITENTRIEBBILDUNG UND/ODER GESTEUERTER ABSCISSIONSZONENBILDUNG**
PLANTS WITH CONTROLLED SIDE-SHOOT FORMATION AND/OR CONTROLLED ABSCISSION AREA FORMATION
PLANTES AVEC FORMATION CONTROLEE DE POUSSES LATERALES ET/OU FORMATION CONTROLEE DE ZONES D'ABSCISSION

(30) Priorität: 15.04.1997 DE 19715700
(43) Veröffentlichungstag der Anmeldung: 26.01.2000
(73) Patentinhaber: Theres, Nikolaus, 50259 Pulheim (DE)
(72) Erfinder: Theres, Nikolaus, 50259 Pulheim (DE)
(74) Vertreter: Dehmel, Albrecht, Dr.
(86) Internationale Anmeldenummer: PCT/DE1998/001070
(87) Internationale Veröffentlichungsnummer: WO 1998/046759

(56) Entgegenhaltungen:
- MANDEL A. AND YANOFSKY M.: "A gene triggering flower formation in Arabidopsis" NATURE, Bd. 377, 12. Oktober 1995, Seiten 522-524, XP002081234
- SZYMKOWIAK E J ET AL: "Effect of lateral suppressor on petal initiation in tomato." PLANT JOURNAL, (1993 JUL) 4 (1) 1-7. JOURNAL CODE: BRU. ISSN: 0960-7412., XP002081235 in der Anmeldung erwähnt
- SCHUMACHER K. ET AL.: "Genetic and physical mapping of the lateral suppressor (ls) locus in tomato." MOLECULAR AND GENERAL GENETICS, (1995 MAR 20) 246 (6) 761-6. JOURNAL CODE: NGP. ISSN: 0026-8925., XP002081236 in der Anmeldung erwähnt
- VAN DER KROL A.R. ET AL: 'Flavonoid genes in petunia: addition of a limited number of gene copies may lead to a suppression of gene expression' THE PLANT CELL Bd. 2, April 1990, Seiten 291 - 299
- SCHUMACHER K. ET AL: 'The lateral suppressor (Ls) gene of tomato encodes a new member of the VHIID protein family' PNAS Bd. 96, Januar 1999, Seiten 290 - 295
- SAMBROOK J. ET AL: 'Molecular Cloning', 1989, COLD SPRING HARBOR LABORATORY PRESS, USA
- HAMES B.D. ET AL: 'Nuclec acid hybridisation: a practical approach', 1985, IRL PRESS, OXFORD ENGLAND
- NAPOLI C. ET AL: 'Introduction of a chimeric chalcone synthase gene into petunia results in reversible co-suppression of homologous genes in trans' THE PLANT CELL Bd. 2, April 1990, Seiten 279 - 289

## Beschreibung

Die Erfindung betrifft Desoxyribonukleinsäure die Polypeptide codieren, die für die Steuerung der Seitentriebbildung und Petalenbildung und Abscissionszonenbildung verantwortlich sind, sowie die durch die Desoxyribonukleinsäure codierten Polypeptide und Aminosäuresequenzen. Die Erfindung betrifft weiterhin Pflanzen mit gesteuerter Seitentriebbildung und Petalenbildung und gesteuerter Bildung von Abscissionszonen, wobei die für die Seitentriebbildung und Petalenbildung und Abscissionszonenbildung verantwortliche exprimierbare Desoxyribonukleinsäure oder deren Fragment stabil in das Genom der Pflanzenzelle oder das Pflanzengewebe integriert ist. Die Erfindung betrifft ferner Verfahren zur Herstellung von Pflanzen mit gesteuerter Seitentriebbildung und Petalenbildung und gesteuerter Bildung von Abscissionszonen, wobei die für die Seitentriebbildung und Petalenbildung und Abscissionszonenbildung verantwortliche exprimierbare Desoxyribonukleinsäure oder deren Fragment stabil in das Genom von Pflanzenzellen oder Pflanzengeweben integriert wird und die erhaltenen Pflanzenzellen oder Pflanzengewebe zu Pflanzen regeneriert werden. Darüberhinaus betrifft die Erfindung Pflanzen sowie das Saatgut von Pflanzen, die nach dem erfindungsgemäßen Verfahren erhältlich sind.

### Technischer Hintergrund

Die Leistungseigenschaften von Nutz- und Zierpflanzen werden maßgeblich durch ihre Architektur bestimmt. Während sich der Grundbauplan einer Pflanze in der Embryonalentwicklung manifestiert, ist die postembryonale Phase durch die Aktivität der Apikalmeristeme gekennzeichnet. Von zentraler Bedeutung ist dabei die Fähigkeit des Sproßapikalmeristems (SAM) höherer Pflanzen, Verzweigungen des Sprosses zu initüeren und deren Enwicklung zu steuern. So wird der Habitus einer Pflanze und damit ein wesentliches Leistungsmerkmal geprägt durch Zahl, Anordnung und Entwicklungsintensität ihrer Seitentriebe. Die Verzweigung des Sprosses kann sowohl terminal als auch lateral erfolgen. Die terminale Verzweigung, bei der sich das SAM in zwei Teile aufspaltet, tritt vor allem bei niederen Kormophyten auf und ist nur für wenige Blütenpflanzen beschrieben worden (Steeves und Sussex, 1989, Patterns in Plant Development, 2. Auflage, Cambridge University Press, Cambridge). Die für Blütenpflanzen typische laterale Verzweigung beruht auf der Entstehung neuer Sproßapikalmeristeme in den Blattachseln. Diese gehen auf Zellen des SAM zurück, deren meristematischer Charakter im Gegensatz zu den sie umgebenden Zellen, die an der Entwicklung der Blattprimordien beteiligt sind, erhalten bleibt. Im weiteren Entwicklungsverlauf bildet sich aus diesen Restmeristemen eine Seitenknospe, die neben einigen Blattprimordien ein Apikalmeristem enthält. dessen Aktivität jedoch der Kontrolle durch das SAM des Hauptsprosses unterliegt.

Die Analyse von Pflanzenmutanten ergab, daß die Verzweigung des Sproßsystems von genetischen Faktoren gesteuert wird. So ist zum Beispiel bei der Tomate (*Lycopersicon esculentum*) eine Reihe von Mutanten beschrieben worden, deren Seitentriebbildung auf unterschiedlichen Stadien gehemmt ist (z.B. *blind, blind-2, torosa, lateral suppressor).* Eine morphologische Charakterisierung zeigte, daß bei den Tomatenmutanten *blind, blind-2* und *torosa* das Austreiben der Achselknospen gestört ist (Tucker, 1979, Ann. Bot. 43:571-577; Mapelli und Lombardi, 1982, Plant & Cell Physiol. 23:751-757). Dagegen unterbleibt bei Pflanzen, die homozygot für die rezessive *lateral suppressor (ls)* Mutation sind, bereits die Anlage der meisten Seitenknospen (Brown, 1955, Rep. Tomato Genetics Cooperative, 5:6-7). Eine histologische Analyse (Malayer und Guard, 1964, Amer. Jour. Bot., 51:140-143) zeigt, daß direkt vom SAM abstammende Zellen in den Achseln der Blattprimordien, auf deren meristematischer Aktivität die Entstehung der Seitenknospen beruht, in der *lateral suppressor*-Mutante fehlen. Würde ein Fehlen von Seitentrieben in allen Blattachseln zu einer Termination der Sproßachse in der ersten Infloreszenz führen, so zeigt sich mit dem Übergang zur floralen Entwicklung, daß die Fähigkeit zur Anlage von Achselmeristemen in der Mutante nicht vollständig verloren gegangen ist. In der Achsel des unmittelbar vor der Infloreszenz angelegten Blattprimordiums wird auch in homozygoten *ls*-Mutanten häufig ein Meristem angelegt. Die Anlage dieses für den sympodialen Aufbau der Sproßachse notwendigen Meristems wird oft von der Entstehung einer Seitenknospe in der Achsel des nächstälteren Blattes begleitet. Nach dem Übergang in die florale Phase ist die Entwicklung der *ls*-Mutante durch eine geringere Anzahl von Blüten pro Infloreszenz (Williams, 1960, Heredity, 14:285-296), die fehlende Anlage von Petalenprimordien (Szymkowiak und Sussex, 1993, Plant J., 4:1-7) und eine aberrante Zahl von Stamina und Karpellen (Groot *et al.,* 1994, Sci. Hort., 59:157-162) gekennzeichnet. Weiterhin wird bei der Mutante eine reduzierte Fertilität beobachtet, was auch eine Reduktion des Ertrages zur Folge hat und der Grund dafür ist, daß die *ls*-Mutante für den Ertragsanbau keine Bedeutung erlangte.

Eine weitere phänotypische Veränderung der *ls*-Mutante betrifft die Ausbildung von Abscissionszonen in den Blüten- und Fruchtstielen. Während Wildtyppflanzen einen Bereich von 5-10 Lagen von kleineren Zellen besitzen, an dessen distalem Ende die nicht befruchtete Blüte bzw. die reife Frucht sich von der Pflanze löst (Roberts et al. 1984, Planta, 160: 159-163), wird diese Abscissionszone in der *ls*-Mutante nicht ausgebildet und die Frucht löst sich bei der Ernte ohne Reste des Fruchtstiels und der Kelchblätter von der Pflanze.

Die beobachteten phänotypischen Veränderungen sind auf physiologischer Ebene mit Störungen in den Gleichgewichten bestimmter Pflanzenhormone korreliert. Im Vergleich zum Wildtyp wurden in den Sproßspitzen von *ls*-Mutanten niedrigere Cytokininkonzentrationen gemessen (Maldiney *et al.,* 1986, Physiol. Plant, 68:426-430; Sossountzov *et al.,* 1988, Planta, 175:291-304), während die Mengen β-Indolylessigsäure(LAA)-ähnlicher Verbindungen sowie Gibberellin- und Abscisinsäure deutlich erhöht sind (Tucker, 1976, New Phytol., 77:561-568). Versuche, die Defekte der *ls*-Mutante durch Einfuhrung eines Isopentenyltransterase-Gens aus *Agrobacterium tumefaciens* zu beheben, führten zwar zu einer Erhöhung der endogenen Cytokininkonzentrationen, aber nicht zu einer Normalisierung der Seitentriebentwicklung (Groot *et al.,* 1995, Plant Growth Regulation, 16:27-36).

Aufgrund des großen Interesses der Züchter an einstämmigen Tomatensorten gab es schon frühzeitig Bestrebungen, die *l*s-Mutante für den gewerblichen Anbau nutzbar zu machen. Da die DNA-Sequenz des für die Seitentriebbildung und/oder Petalenbildung und/oder Abscissionszonenbildung verantwortlichen Gens (*Ls*-Gen) bisher nicht bekannt war, wurde wiederholt versucht, auf genetischem Wege die erwünschten Effekte auf die Seiteutriebbildung von den unerwünschten Effekten auf Fertilität und Ertrag zu trennen. Alle diese Bemühungen führten bis jetzt jedoch nicht zum Erfolg.

Zur Isolierung von Genen, die nur durch einen mutanten Phänotyp und ihre Position auf der genetischen Karte charakterisiert sind, wurden in den letzten Jahren bevorzugt die Strategien der Insertionsmutagenese und der positionellen Klonierung verwendet. Die Insertionsmutagenese nutzt durch Insertion einer bekannten Sequenz erzeugte mutante Allele zur Isolierung der auf diese Weise molekular markierten Gene. Bei Pflanzen wurden sowohl die T-DNA von *Agrobacterium tumefaciens* (Koncz *et al.,* 1992, Plant Mol. Biol., 20:963-976) als auch transponierbare Elemente (Gierl und Saedler, 1992, Plant Mol. Biol., 19: 39-49) zur Insertionsmutagenese eingesetzt (Jones *et al.,* 1994, Science 266:789-793). Da die transponierbaren Elemente *Ac* und *Ds* aus Mais präferentiell an gekoppelte Positionen auf dem gleichen Chromosom transponieren *(Knapp et al.,* 1994, Mol. Gen. Genet., 243:666-673), ist eine Transposonmutagenese dann besonders aussichtsreich, wenn eine Ausgangslinie zur Verfügung steht, in der das transponierbare Element in enger Kopplung zu dem gesuchten Gen vorliegt. Da eine solche Tomatenlinie nicht zur Verfügung steht, ist eine Transposonmutagenese zur Isolierung des *Ls*-Gens wenig aussichtsreich.

Die Strategie der positionellen Klonierung wurde zur Analyse der molekularen Grundlagen von Erbkrankheiten in Säugetieren entwickelt und u.a. für die Isolierung der menschlichen Gene für Duchenne Muskeldystrophie (Koenig *et al.,* 1987, Cell, 50:509-517), Cystische Fibrose (Rommens *et al.,* 1989, Science, 245:1059-1065) und Chorea-Huntington (Huntington's Disease Research Group, 1993, Cell, 72:971-983) eingesetzt. In Zeichnung 1 ist der Ablauf einer positionellen Klonierung schematisch dargestellt. Von zentraler Bedeutung für diese Strategie ist die Integration des klassischen genetischen Locus in eine Karte molekularer Marker. Der Einsatz von Restriktionsfragmentlängen-Polymorphismen (RFLP) als genetische Marker (Botstein *et al.,* 1980, Am. J. Hum. Genet., 32:314-331) ermöglicht die Identifizierung enggekoppelter DNA-Fragmente aus der Umgebung des zu isolierenden Gens. Diese Fragmente dienen anschließend als Hybridisierungssonden in der Southern-Analyse mittels Pulsfeldgelelektrophorese (Chu *et al.,* 1986, Science, 234:1582-1585) aufgetrennter hochmolekularer DNA, um den relativen genetischen Abstand in einen absoluten Wert für den physikalischen Abstand zu übersetzen, der durch den sogenannten "chromosome walk" zu überbrücken ist. Ausgehend von den flankierenden Markern als Startpunkte wird die Umgebung des gesuchten Gens in Form überlappender DNA-Fragmente isoliert. Je nach Distanz der flankierenden Marker in der genetischen Karte handelt es sich bei den DNA-Fragmenten um YAC- oder Cosmid-Klone (Burke *et al.,* 1987, Science, 236:806-812). RFLP-Karten hoher Markerdichte sind von Nam *et al.,* 1989, Plant Cell, 1:699-705, und Tanksley *et al.*, 1992, Genetics, 132:1141-1160, entwickelt worden. Grill und Somerville, 1991 , Mol. Gen. Genet., 226:484-490, und Martin *et al.,* 1992, Mol: Gen. Genet., 233:25-32, beschreiben die Erstellung von YAC-Bibliotheken.

In der klassischen genetischen Karte der Tomate kartiert der *Ls*-Locus auf dem langen Arm von Chromosom 7 (Taylor und Rossall, 1982, Planta, 154:1-5). Schumacher *et al.,* 1995, Mol. Gen. Genet., 246:761-766, beschreiben eine Integration des *Ls*-Locus in die RFLP-Karte wobei der *Ls*-Locus In einem 0,8 cM-Intervall nahe dem distalen Ende von Chromosom 7 kartiert wurde. Weiterhin beschreiben Schumacher *et al.,* daß der *Ls*-Locus durch die RFLP-Marker CD61 und CD65 begrenzt wird. Die physikalische Kartierung mittels Pulsfeldgelelektrophorese zeigte, daß CD61 und CD65 nicht mehr als 375 kb voneinander entfernt liegen

Bei vielen Nutzpflanzen ist die Bildung von Seitentrieben vom Standpunkt der landwirtschaftlichen Nutzung her aus verschiedenen Gründen unerwünscht:
1. Die jungen Seitentriebe sind zunächst "Sink"-Organe (Verbraucher-Organe) und reduzieren daher den Ertrag am Hauptsproß.
2. Stark verzweigte Sproßsysteme stellen für eine mechanische Bearbeitung (z.B. maschinelle Ernte) oft ein kaum überwindliches Hindernis dar.

Aus diesen Gründen ist schon frühzeitig versucht worden, seitentriebfreie Sorten auf konventionellem Wege zu züchten. Bei einzelnen Nutzpflanzen (z.B. Sonnenblume) ist dies gelungen. Dagegen ist bei vielen anderen dikotylen Nutzpflanzen (z.B. Tomate, Gurke, Apfelbaum. Bimbaum) die Einstämmigkeit zwar wünschenswert, konnte aber bisher nicht in leistungsfähigen Zuchtsorten realisiert werden. Auch bei monokotylen Nutzpflanzen wie Mais und Zuckerrohr ist die Unterdrückung der Seitentriebbildung von Vorteil und für die gewerbliche Nutzung sehr erwünscht. Zur Zeit wird bei dem in Mittel- und Nordeuropa üblichen Gewächshausanbau z.B. der Tomate die Einstämmigkeit dadurch erreicht, daß die Seitentriebe manuell entfernt werden. Da das Entfernen der Seitentriebe nicht maschinell ausgeführt werden kann, ist dies mit einem hohen Kostenaufwand verbunden. Außerdem sind die Pflanzen an den Wundstellen für Infektionen durch Pathogene wie pathogene Bakterien, Viren und Pilze sehr empfänglich, weshalb die Entfernung der Seitentriebe häufig zu einer Ausbreitung von Krankheiten im Gewächshaus beiträgt.

Dagegen ist bei vielen Zierpflanzen die zusätzliche Ausbildung von Seitentrieben und als Folge davon eine vermehrte Blütenbildung erwünscht. Die vermehrte Seitentriebbildung ist ebenfalls bei verschiedenen Nutzpflanzen wie Kartoffeln, dem Kaffee- oder Teestrauch von hohem Nutzen. Es besteht somit ein Bedarf an kostengünstigen, leistungsfähigen Nutzpflanzen und Zierpflanzen, in denen die Seitentriebbildung verstärkt oder unterdrückt ist.

Eine Inhibition der Ausbildung von Abscissionszonen ist in einer Reihe von Pflanzen von Interesse. So führt der vorzeitige Abwurfvon Früchten bei Zitruspflanzen zu Ertragsverlusten, die sich verhindern ließen, wenn keine Abscissionszonen ausgebildet würden. Ähnliches findet sich auch bei anderen Obstsorten wie Kirschen, Pfirsichen oder Johannisbeeren. Ferner ist eine Inhibition der Ausbildung von Abscissionszonen z.B. in der Tomatenpflanze von Vorteil. Werden die Abscissionszonen nicht ausgebildet, löst sich die Frucht bei der Ernte ohne Reste des Fruchtstiels und der Kelchblätter von der Pflanze. Diese Eigenschaft ist erwünscht, wenn die Tomaten maschinell geerntet und anschließend zu Produkten wie Tomatenmark verarbeitet werden, da die Kelchblätter und Fruchtstiele die Qualität der Tomatenprodukte verschlechtern.

Eine verstärkte Ausbildung von Abscissionszonen kann bei Zierpflanzen dazu genutzt werden, daß die Blüten nach dem Abblühen von selbst abfallen und nicht wie bei vielen Balkon- und Gartenpflanzen manuell entfernt werden müssen. Falls dies unterbleibt, wird oft die Bildung neuer Blüten unterdrückt.

### Kurze Beschreibung der Erfindung

Die Isolierung und Klonierung des *Ls*-Gens würde die Möglichkeit eröffnen, die Aktivität dieses Gens gezielt zu verändern und damit in transgenen Pflanzen die Anlage von Seitentrieben entweder zu unterdrücken oder zu verstärken. Ferner wird die Möglichkeit eröffnet, durch gezielte Veränderung der Aktivität des *Ls*-Gens die Bildung von Abscissionszonen und/oder Petalen zu unterdrücken oder zu verstärken. Der Erfindung liegt daher die Aufgabe zugrunde, das *Ls*-Gen oder ein DNA-Fragment, das dieses Gen enthält, zu isolieren, seine Sequenz zu bestimmen und ein Verfahren zur Erzeugung von transgenen Pflanzen bereitzustellen, in denen die Aktivität des *Ls*-Gens gezielt verändert wurde, um die Anlage von Seitentrieben und/oder die Bildung von Abscissionszonen und/oder Petalen entweder zu unterbinden oder zu'verstärken.

Die Aufgabe der Erfindung wird durch die Bereitstellung von Desoxyribonukleinsäure mit einer Nukleotidsequenz gemäß SEQ ID NO:1, 9 oder 13 und von Nukleinsäuren, die mit der Nukleotidsequenz gemäß SEQ ID NO:1, 9 oder 13 hybridisieren, gelöst, wobei die Desoxyribonukleinsäure mit einer Nukleotidsequenz gemäß SEQ ID NO:1, 9 oder 13 und die Nukleinsäuren, die mit der Nukleotidsequenz gemäß SEQ ID NO:1, 9 oder 13 hybridisieren, Polypeptide codieren, die für die Steuerung der Seitentriebbildung und Petalenbildung und Abscissionszonenbildung verantwortlich sind. Der Ausdruck "Hybridisierung" bezieht sich erfindungsgemäß auf konventionelle Hybridisierungsbedingungen, bevorzugt bezieht sich "Hybridisierung" auf solche Hybridisierungsbedingungen, bei denen der T_{M}-Wert im Bereich von T_{M} -Wert 45°C bis T_{M} - Wert 68°C liegt. Besonders bevorzugt bezieht sich der Begriff "Hybridisierung" auf stringente Hybridisierungsbedingungen. Ferner betrifft die Erfindung die durch die Nukleotidsequenzen codierten Polyeptide und Aminosäuresequenzen.

Eine weitere Aufgabe der Erfindung wird durch ein Verfahren zur Herstellung von Pflanzen mit gesteuerter Seitentriebbildung und Petalenbildung und Abscissionszonenbildung gelöst, wobei die für die Steuerung der Seitentriebbildung und Petalenbildung und Abscissionszonenbildung verantwortliche exprimierbare Desoxyribonukleinsäure oder deren Fragment stabil in das Genom von Pflanzenzellen oder Pflanzengeweben integriert wird und die erhaltenen Pflanzenzellen oder Pflanzengewebe zu Pflanzen regeneriert werden.

In der vorliegenden Erfindung wird ein Verfahren bevorzugt, bei dem die integrierte DNA die Seitentriebbildung und Petalenbildung und Abscissionszonenbildung unterdrückt. Besonders bevorzugt ist ein Verfahren bei dem die integrierte DNA in Antisense-Orientierung zur komplementären, die Seitentriebbildung und Petalenbildung und Abscissionszonenbildung steuernden, endogenen Sequenz exprimiert wird. Ebenfalls besonders bevorzugt ist ein Verfahren bei dem die integrierte DNA in Sense-Orientierung zur komplementären, die Seitentriebbildung und Petalenbildung und Abscissionszonenbildung steuernden, endogenen Sequenz exprimiert wird. Weiterhin besonders bevorzugt ist ein Verfahren, bei dem die Seitentriebbildung und Petalenbildung und Abscissionszonenbildung durch ein Ribozym, umfassend die erfindungsgemäßen DNA-Sequenzen oder deren Fragment oder Derivat, unterdrückt wird. Weiterhin besonders bevorzugt ist ein Verfahren, bei dem die erfindungsgemäßen DNA-Sequenzen oder deren Fragment oder Derivat eingesetzt wird, um das endogene Gen in Pflanzen durch homologe Rekombination auszuschalten ("knock-out").

In der vorliegenden Erfindung wird ferner ein Verfahren bevorzugt, bei dem die ins Genom der Pflanzen integrierte DNA die Seitentriebbildung und Petalenbildung und Abscissionszonenbildung verstärkt. Besonders bevorzugt ist ein Verfahren, bei dem die erfindungsgemäße DNA in Sense-Orientierung zur für die Steuerung der Seitentriebbildung und Petalenbildung und Abscissionszonenbildung verantwortlichen endogenen Sequenz exprimiert wird.

Insbesondere bevorzugt ist das erfindungsgemäße Verfahren zur Herstellung von transgenen Tomaten-, Raps-, Kartoffel- oder Löwenmaulpflanzen. Insbesondere bevorzugt ist auch ein .erfindungsgemäßes Verfahren zur Herstellung von transgenen Pflanzen, wobei die ins Genom der Pflanzen integrierte DNA die Sequenz gemäß SEQ ID NO:1, 9 oder 13 oder deren Fragment umfaßt, oder komplementär zu dieser Sequenz oder deren Fragment ist, oder mit der Sequenz gemäß SEQ ID NO:1, 9 oder 13 oder deren Fragment hybridisiert und ein Polypeptid mit der biologischen Aktivität der Seitentriebbildung und Petalenbildung und Abscissionszonenbildung codiert.

Die Erfindung betrifft weiterhin transformierte Pflanzenzellen oder transformiertes Pflanzengewebe, bei dem eine für die Kontrolle der Seitentriebbildung und Petalenbildung und Abscissionszonenbildung verantwortliche exprimierbare Desoxyribonukleinsäure oder deren Fragment stabil in das Genom der Pflanzenzelle oder das Pflanzengewebe integriert ist. Die Erfindung betrifft ferner Pflanzen sowie das Saatgut von Pflanzen, die nach dem erfindungsgemäßen Verfahren erhältlich sind.

Die Erfindung wird weiter durch die folgenden Zeichnungen erläutert.

In Zeichnung 1 ist der Ablauf einer positionellen Klonierung schematisch dargestellt.

In Zeichnung 2 ist (a) ein Ausschnitt aus der von Tanksley *et* al., 1992, Genetics, 132:1141-1160 veröffentlichten RFLP-Karte dargestellt. In (b) ist die *Ls*-Region nach Schumacher *et al.,* 1995, Mol. Gen. Genet., 246:761-766, in diese Karte integriert.

Zeichnung 3 zeigt die Kartierung von cDNA- und Cosmid-Klonen aus der *Ls*-Region. Die Cosmid-Klone A, B, C, D, E, F, G und L sowie der YAC-Klon CD61-5 sind durch Balken symbolisiert. Die Positionen der cDNA-Klone c10, c21, y25 und ET sind durch offene Rechtecke dargestellt. Die gestrichelten Linien symbolisieren Rekombinationspunkte in den F2-Pflanzen 23, 24, 865 und 945.

Zeichnung 4 zeigt die Autoradiographie einer Southern-Blot-Analyse zum Nachweis von *Ls*-verwandten Genen in verschiedenen Pflanzenarten. Genomische DNA aus Tomate *(Lycopersicon esculentum),* Kartoffel *(Solanum tuberosum)* und Löwenmaul *(A. majus)* wurde mit dem Restriktionsenzym EcoRI behandelt und mit dem cDNA-Klon ET hybridisiert.

In Zeichnung 5 ist die Nukleotidsequenz und die davon abgeleitete Aminosäuresequenz (Ein-Buchstaben-Code) des Wildtyp *Ls*-Gens aus der Tomate (*Lycopersicon esculentum*) aufgeführt.

In Zeichnung 6 ist die Nukleotidsequenz und die davon abgeleitete Aminosäuresequenz (Ein-Buchstaben-Code) des *Ls*-homologen Gens aus Kartoffel *(Solanum tuberosum)* aufgeführt.

In Zeichnung 7 ist die Nukleotidsequenz und die davon abgeleitete Aminosäuresequenz (Ein-Buchstaben-Code) eines 687 bp langen DNA-Fragments des *Ls*-homologen Gens aus *Arabidopsis thaliana* aufgeführt.

Zeichnung 8 zeigt eine Aminosäuresequenz-Gegenüberstellung (engl.: *alignment)* der *Ls*-Polypeptide aus *Arabidopsis thaliana* (LsAt), *Lycopersicon esculentum* (LsLe) und *Solanum tuberosum* (LsSt). Für die Aminosäuren wurde der Ein-Buchstaben-Code verwendet. Identische Aminosäuren sind schwarz unterlegt, ähnliche Aminosäuren sind mit einer grauen Schattierung untertegt. Ein Strich (-) bedeutet fehlende Sequenzinformation, ein Punkt (.) bedeutet eine zusätzliche Aminosäure in einem Polypeptid. Ein Stern (*) bedeutet ein Stopcodon auf Nucleinsäureebene.

### Detaillierte Beschreibung der Erfindung

Die Methode der Klonierung mehrerer hundert Kilobasen großer DNA-Fragmente als künstliche Hefe-Chromosomen (Yeast Artificial Chromosome: YAC) in *Saccharomyces cerevisiae* (Burke *et al.,* 1987, Science, 236:806-812) erlaubt die Umsetzung der physikalischen Karte in eine Reihe überlappender YAC-Klone, die das zu isolierende Gen überspannen. Aus einer YAC-Bibliothek der Tomate (Martin *et al.,* 1992, Mol. Gen. Genet., 233:25-32) wurden Klone isoliert, die den RFLP-Marker CD61 enthielten. Durch Kartierung der YAC-Endfragmente relativ zu den das *Ls*-Gen flankierenden RFLP-Markem sowie zu den Rekombinationsbruchpunkten und zum *Ls*-Gen selbst wurde die Lage der isolierten DNA-Fragmente in der-*Ls*-Region bestimmt. Dabei stellte sich heraus, daß der YAC-Klon CD61-5 sowohl mit CD61 als auch mit CD65 hybridisierte und somit den gesamten genomischen Abschnitt einschließlich des *Ls*-Gens enthält. In Zeichnung 3 ist die Lage der Marker und des YAC-Klons schematisch dargestellt.

Zur Identifizierung von innerhalb dieses YAC-Klons gelegenen kodierenden Regionen wurde dieser Klon als radioaktiv markierte Sonde zur Durchmusterung einer cDNA-Bibliothek (Simon, 1990, Dissertation, Universität zu Köln, Köln, FRG) eingesetzt. Die verwendete cDNA-Bibliothek ist aus RNA sowohl aus vegetativen als auch aus floralen Sproßspitzen hergestellt worden und repräsentiert somit die exprimierten Gene der Gewebe, in denen sich der Phänotyp der *ls*-Mutation manifestiert. Eine Charakterisierung der cDNA-Klone durch kreuzweises Hybridisieren ergab, daß die gereinigten Klone insgesamt 29 verschiedene Transkripte repräsentierten. Bei der anschließenden Feinkartierung der cDNA-Klone relativ zu den Rekombinationsbruchpunkten im Intervall CD61-CD65 stellte sich heraus, daß nur der cDNA-Klon y25 mit dem *Ls*-Gen kosegregierte und als Kandidat für dieses Gen in Frage kam. Im Anschluß an die Etablierung eines Cosmid-"Contigs" wurden auch Cosmid-Klone als Sonden eingesetzt, um weitere cDNA-Klone aus dem CD61-CD65-Intervall zu isolieren, die beim Durchmustern mit dem YAC-Klon CD61-5 als Sonde aufgrund der hohen Komplexität der Sonde nicht nachweisbar waren. In diesen Experimenten wurden drei weitere cDNA-Klone (c10, c21 und ET) isoliert, die ebenfalls mit dem *Ls*-Gen kosegregierten und als weitere Kandidaten für das *Ls*-Gen in Frage kamen. Es wurden somit insgesamt vier cDNA-Klone aus der *Ls*-Region identifiziert, die Kandidaten für das *Ls-Gen* waren. Die Klone sind in Zeichnung 3 durch offene Rechtecke dargestellt.

Um das *Ls*-Gen zusammen mit den für die Steuerung der Expression notwendigen Promotorsequenzen zu klonieren, wurde der cDNA-Klon y25 als Ausgangspunkt für die Isolierung von kürzeren genomischen DNA-Fragmenten aus der *Ls*-Region benutzt. Zu diesem Zweck wurde eine genomische Cosmid-Bibliothek der Tomate in dem Vektor pCLD04541 (Bent *et al.,* 1994, Science, 265:1856-1860) etabliert. Dieser Vektor enthält die zur Pflanzeutransformation notwendigen T-DNA-"border"-Sequenzen und erlaubt daher eine Einführuug der isolierten DNA-Fragmente in Pflanzenzellen ohne weitere Klonierungsschritte. Aus dieser Bibliothek wurde in mehreren üblichen Klonierungsschritten eine Reihe von überlappenden Cosmid-Klonen isoliert. Die Kartierung dieser Cosmid-Klone relativ zu den Rekoxnbinationsbmchpunkten im untersuchten Intervall zeigte, daß die isolierten genomischen DNA-Fragmente zusammen einen genomischen Abschnitt von ca. 60 kb überspannen. Die Lage der Cosmid-Klone ist in Zeichnung 3 schematisch dargestellt.

Zur Untersuchung der Frage, ob ein Gen aus dem als Cosmid-"Contig" isolierten genomischen DNA-Abschnitt die in der *ls*-Mutante fehlende biologische Funktion zur Anlage von Seitentrieben, Petalen und Abscissionszonen ersetzen kann (Komplementationsexperiment), wurde die *ls*-Mutante mit den Cosmid-Klonen A, B, C, D, E, F, G und L transformiert. Bei allen Transgenen, die durch Einführung der Cosmide A, B, C, D, E und F hergestellt wurden, konnte keine Veränderung des Phänotyps beobachtet werden. Dagegen war bei acht unabhängigen transgenen Pflanzen, die entweder das Cosmid G oder L enthielten, eine partielle oder vollständige Wiederherstellung des Wildtyp-Phänotyps, zu beobachten. Die Ergebnisse der Komplementationsexperimente sind in Tabelle I aufgeführt.

**Tabelle 1:**

| Versuche zur Komplementation der *ls*-Mutante durch Cosmid-Transformation | | |
|---|---|---|
| Cosmid | Zahl der transformierten Pflanzen | Zahl der komplementierten Pflanzen |
| pCLDO4541 | 8 | 0 |
| A | 5 | 0 |
| B | 15 | 0 |
| C | 5 | 0 |
| D | 7 | 0 |
| E | 2 | 0 |
| F | 8 | 0 |
| G | 5 | 3 |
| L | 11 | 5 |

Diese transgenen Pflanzen bilden während der vegetativen Entwicklung Seitentriebe und in der Blütenentwicklung wieder Petalen und Abscissionszonen. Eine Southern-Blot-Analyse der transgenen Cosmid G oder Cosmid L enthaltenden Pflanzen ergab, daß bei Pflanzen, die keine Komplementation zeigten, die T-DNA nur unvollständig übertragen worden war. Somit ist gezeigt worden, daß die eingeführten DNA-Fragmente die in der Mutante nicht vorhandene genetische Information zur Anlage von Seitentrieben, Petalen und Abscissionszonen komplementieren können.

Aufgrund von Komplementationsexperimenten mit Subfragmenten des Cosmids G ließ sich der DNA-Abschnitt, in dem das *Ls*-Gen liegt, genauer bestimmen. Während nach Transformation mit DNA-Fragmenten, die das zuvor identifizierte Gen c21 enthielten, keine Komplemetation des *ls*-Phänotyps beobachtet wurde, konnte durch Einführung eines ca. 6 Kb-Fragments, das das Gen ET trägt, in acht unabhängigen transgenen Pflanzen der Wildtyp-Phänotyp) wiederhergestellt werden. Eine DNA-Sequenzanalyse ergab, daß das ET-Gen der *ls*¹-Mutante eine Deletion von 1550 Bp trägt, die die ersten 185 Aminosäuren des Proteins und 865 Bp der davor gelegenen Sequenz entfernt. Ein zweites unabhängiges mutantes Allel, *ls*^{*2*}*,* enthält ein 3 Bp-Insertion und mehrere Punktmutationen in einem kurzen DNA-Abschnitt, von denen eine zu einem Abbruch des Proteins nach 24 Aminosäuren führt. Aus den Komplementationsexperimenten und der Isolierung und Kartierung der cDNAs, sowie den Sequenzanalysen des Gens ET aus dem Wildtyp und zwei unabhängigen *ls*-Allelen ergab sich, daß der cDNA-Klon ET die gesamte codierende Sequenz der mRNA des *Ls*-Gens repräsentiert.

Zur Untersuchung der Frage, ob ähnliche oder homologe Gene auch in anderen Pflanzenarten vorkommen, wurde der cDNA-Klon ET als Hybridisierungssonde in Southern-Experimenten unter reduzierter Stringenz eingesetzt. Der Ausdruck "Pflanze" umfaßt, wie er hier verwendet wird, monokotyle und dikotyle Nutz- und Zierpflanzen. Der Begriff der "reduzierten Stringenz" betrifft hier übliche Hybridisierungsbedingungen mit der Abweichung, daß die Hybridisierungstemperatur zwischen 50°C und 55°C lag. In Kartoffel *(Solanum tuberosum)* und Löwenmaul *(Antirrhinum majus)* konnten mehrere DNA-Fragmente nachgewiesen werden. Aus Löwenmaul wurden durch Hybridisierung bei 55°C mehrere genomische Klone isoliert. Eine DNA-Sequenzanalyse ergab, daß der isolierte Löwenmaul-Klon signifikante Sequenzbomologien zum *Ls*-Gen besitzt. Homologe Gene zum *Ls*-Gen der Tomate können demnach unter Verwendung des cDNA-Klons ET als Sonde nach üblichen Verfahren isoliert werden. Mit genspezifischen Primern wurde mittels PCR aus genomischer DNA von Kartoffel *(Solanum tuberosum)* das *Ls*-homologe Gen isoliert. Das *Ls*-homologe Gen der Kanoffel zeigt zum *Ls*-Gen der Tomate sowohl auf DNA-Ebene als auch auf Proteinebene eine Sequenzidentität von etwa 98 %. Mit degenerierten Primem wurde mittels PCR aus genomischer DNA von Arabidopsis *(Arabidopsis thaliana)* ein 687 bp DNA-Fragment des *Ls*-homologen Gens isoliert. Auf DNA-Ebene zeigt das *Arabidopsis thaliatia*-DNA-Fragment zum *Ls*-Gen der Tomate eine Sequenzidentität von etwa 63 %. Auf Protein-Ebene sind etwa 55 % der Aminosäuren identisch.

Die vorliegende Erfindung betrifft ferner DNA-Sequenzen, die aus einem pflanzlichen Genom stammen und ein Protein kodieren, das zur Steuerung der Seitentriebbildung und/oder Bildung von Petalen und/oder Bildung von Abscissionszonen notwendig ist. Die in der Nukleotidsequenz enthaltene Information fuhrt bei Einfiibrung und Expression in pflanzlichen Zellen zur Bildung einer Ribonukleinsäure. Über diese Ribonukleinsäure kann eine Proteinaktivität in die Zellen eingeführt oder eine endogene Proteinaktivität unterdrückt werden. Besonders bevorzugt ist eine DNA-Sequenz gemäß SEQ ID NO: 1 aus *Lycopersicon* *esculentum,* die in Zeichnung 5 dargestellt ist, eine DNA-Sequenz gemäß SEQ ID NO:9 aus *Solanum tuberosum,* die in Zeichnung 6 dargestellt ist und eine DNA-Sequenz gemäß SEQ ID NO:13 aus *Arabidopsis thaliana,* die in Zeichnung 7 dargestellt ist.

Darüber hinaus betrifft die vorliegende Erfindung die Verwendung der erfindungsgemäßen DNA-Sequenzen oder Fragmente oder Derivate, die durch Insertion, Deletion oder Substitution von diesen DNA-Sequenzen abgeleitet sind, zur Transformation von Pflanzenzellen. Die erfindungsgemäßen DNA-Sequenzen können unter Verwendung verschiedener Verfahren eingesetzt werden, um die Bildung von Seitentrieben und somit Verzweigungen des Sproßsystems und/oder Petalen und/oder Abscissionszonen zu unterdrücken:
1. Um die Bildung von Seitentrieben und/oder Petalen und/oder Abscissionszonen zu unterdrücken, kann die erfindungsgemäße DNA-Sequenz in Antisense (Gegensinn)- oder Sense (Sinn)-Orientierung in übliche Vektoren (z.B. Plasmide) kloniert werden und dabei mit Steuerelementen für die Expression in pflanzlichen Zellen, wie Promotoren und Terminatoren, kombiniert werden. Mit den hergestellten Vektoren können pflanzliche Zellen transformiert werden mit dem Ziel, die Synthese des endogenen Proteins zu verhindern. Zu diesem Zweck können auch kürzere Teile der erfindungsgemäßen DNA-Sequenz, also Fragmente, oder DNA-Sequenzen mit Sequenzähnlichkeit von 50% bis 100%, also Derivate, verwendet werden. So kann das aus Arabidopsis isolierte *Ls*-homologe Gen z.B. zur Unterdrückung der Bildung von Seitentrieben und somit Verzweigungen des Sproßsystems und/oder Petaleu und/oder Abscissionszonen in der verwandten Art *Brassica napus* (Raps) eingesetzt werden. Die gezielte Unterdrückung einer Genaktivität in pflanzlichen Zellen durch Einführung von Antisense- oder Sense-Konstrukten ist ein übliches Verfahren, das in vielen Fällen erfolgreich eingesetzt worden ist (Gray *et al.,* 1992, Plant. Mol. Biol., 19:69-87).
2. Weiterhin kann die Bildung von Seitentrieben und/oder Petalen und/oder Abscissionszonen durch Expression eines für diesen Zweck konstruierten Ribozyms unter Verwendung der erfindungsgemäßen DNA-Sequenzen inhibiert werden. Die Herstellung und Verwendung von Ribozymen wird in de Feyter *et al.,* 1996, Mol. Gen. Genet., 250:329-338 fiir Tabakmosaikvirus-resistente Tomaten- und Tabakpflanzen beschrieben.
3. Außerdem kann die erfindungsgemäße DNA-Sequenz verwendet werden, um das endogene Gen zu inaktivieren. Anhand der erfindungsgemäßen DNA-Sequenzen können Oligonukleotide synthetisiert werden, um Pflanzen im Rahmen von Mutageneseexperimenten mit Hilfe der PCR-Technik auf die Anwesenheit von Insertionen (z.B. transponierbare Elemente oder die T-DNA von *Agrobacterium tumefaciens)* im *Ls*-Gen zu testen. Durch solche Insertionen wird die Genaktivität in der Regel blockiert (Koes *et al.,* 1995, Proc. Natl. Acad. Sci. USA, 92:8149-8153).
4. Ebenfalls kann die erfindungsgemäße DNA-Sequenz eingesetzt werden, um das endogene *Ls*-Gen durch homologe Rekombination auszuschalten ("knock-out"). Dieses Verfahren wurde bei Mäusen erfolgreich eingesetzt und ist auch für die Anwendung in Pflanzen von Miao und Lam, 1995, Plant. J., 7:359-365 beschrieben worden.

Im Gegensatz zur Tomate und anderen Nutzpflanzen werden bei Zierpflanzen (z.B. Geranien, Fuchsien, Chrysanthemen) häufig Phänotypen bevorzugt, die aufgrund einer starken Entwicklung der Seitentriebe einen buschigen Wuchs zeigen. Um diese Wuchsformen zu erzeugen, werden die Pflanzen heute entweder dekapitiert, was das Austreiben der Seitenachsen fördert, oder mit bestimmten Chemikalien behandelt. Auch diese Praxis ist mit einem beträchtlichen finanziellen Aufwand verbunden. Die erfindungsgemäße Herstellung von transgenen Pflanzen mit buschigen Wuchsformen stellt in diesen Fällen eine kostengünstigere Alternative dar.

Eine verstärkte Ausbildung von Abscissionszonen kann bei Zierpflanzen dazu genutzt werden, daß die Blüten nach dem Abblühen von selbst abfallen und nicht wie bei vielen Balkon- und Gartenpflanzen manuell entfernt werden müssen. Falls dies unterbleibt, wird oft die Bildung neuer Blüten unterdrückt.

Zur Herstellung von transgenen Pflanzen mit starker Seitentriebentwicklung und/oder Abscissionszonenbildung wird die erfindungsgemäße DNA-Sequenz oder deren Fragment oder Derivat, das durch Insertion, Deletion oder Substitution von dieser Sequenz abgeleitet ist, in Sense-Orientierung in Plasmide eingebracht und mit Steuerelementen für die Expression in pflanzlichen Zellen kombiniert. Mit diesen Plasmiden können pflanzliche Zellen transformiert werden, mit dem Ziel der Expression einer translatierbaren Botenribonukleinsäure (mRNA), die die Synthese eines Proteins erlaubt, welches die Bildung und Entwicklung von Seitentrieben und/oder Petalen und/oder Abscissionszonen stimuliert.

Die erfindungsgemäße DNA-Sequenz oder Fragmente oder Derivate, die durch Insertion, Deletion oder Substitution von dieser Sequenz abgeleitet sind, können dazu genutzt werden, aus dem Genom der Tomate oder anderer Pflanzen homologe oder ähnliche DNA-Sequenzen zu isolieren, die ebenfalls die Bildung von Seitentrieben und/oder Petalen und/oder Abscissionszonen beeinflussen. Zu diesem Zweck können die erfindungsgemäße DNA-Sequenz oder Fragmente, z.B. Oligonukleotide, oder Derivate als Sondenmoleküle (engL: *probe)* eingesetzt werden, um nach üblichen Verfahren cDNA-Bibliotheken oder genomische DNA-Bibliotheken der zu durchmusternden Pflanzen abzusuchen. Alternativ dazu können von der erfindungsgemäßen Sequenz degenerierte oder nicht degenerierte Oligonukleotide (Primer) abgeleitet werden, die zur Durchmusterung dieser cDNA-Bibliotheken oder genomischen DNA-Bibliotheken auf PCR-Basis benutzt werden können. Die so isolierten verwandten DNA-Sequenzen können ebenso wie die erfindungsgemäßen DNA-Sequenzen zur Inhibition oder Stimulation der Seitentriebbildung und/oder Petalenbildung und/oder Abscissionszonenbildung bei Pflanzen eingesetzt werden.

Zur Expression der erfindungsgemäßen DNA-Sequenzen in Sense- oder Antisense-Orientierung in pflanzlichen Zellen sind einerseits Transkriptionspromotoren und andererseits Transkriptionsterminatoren notwendig. Promotoren und Terminatoren sind in großer Zahl in der Literatur beschrieben (z.B. Köster-Töpfer *et al.,* 1989, Mol. Gen. Genet., 219:390-6; Rocha-Sosa *et al.,* 1989, EMBO J., 8:23-29). Die transkriptionellen Start- und Terminationsbereiche können entweder aus der Wirtspflanze oder aus einem heterologen Organismus stammen. Die DNA-Sequenzen der Transkriptionsstart- und Transkriptionsterminationsregionen können synthetisch hergestellt oder natürlich gewonnen sein oder eine Mischung aus synthetischen und natürlichen DNA-Bestandteilen enthalten.

Verfahren zur genetischen Modifikation sind sowohl für dikotyle als auch für monokotyle Pflanzen beschrieben worden (Gasser und Fraley, 1989, Science, 244:1293-1299; Potrykus, 1991, Ann. Rev. Plant. Mol. Biol. Plant. Physiol., 42:205-226). Neben der Transformation mit Hilfe *von Agrobacterium tumefaciens* (Hoekema, 1983, Nature, 303:179-180; Filatti *et al.,* 1987, Biotech., 5:726-730) kann DNA durch Transformation von Protoplasten, Mikroinjektion, Elektroporation oder ballistische Methoden in Pflanzenzellen eingeführt werden. Zur Selektion von transformierten Pflanzenzellen wird die einzuführende DNA mit einem Selektionsmarker gekoppelt, der den Zellen eine Resistenz gegenüber Antibiotika (z.B. Kanamycin, Hygromycin, Bleomycin) verleiht. Aus den transformierten Pflanzenzellen können dann in einem üblichen Selektionsmedium ganze Pflanzen regeneriert werden. Die Regenerierung von Pflanzenzellen ist beschrieben, z.B. in der EP-B-0 242 236, auf die hier speziell Bezug genommen wird. Die so erhaltenen Pflanzen werden mit üblichen molekularbiologischen Methoden auf Anwesenheit und Intaktheit der eingeführten DNA getestet. Ist die eingeführte DNA einmal im Genom integriert, so ist sie dort in der Regel stabil und wird an die Nachkommen vererbt. Von den so erhaltenen Pflanzen kann dann mit den üblichen Verfahren Saatgut erhalten werden.

Die folgenden Beispiele erläutern die Erfindung. Die Beispiele sind nicht einschränkend aufzufassen. Wenn nicht anders angegeben wurden molekularbiologische Standardmethoden benutzt, wie von Sambrook *et al.,* 1989, Molecular cloning: A Laboratory Manual. 2. Auflage, Cold Spring Harbor Laboratory Press, Cold Spring Harbor, New York beschrieben. Southem-Hybridisierungen wurden in 6 x SSPE (0,9 M NaCl, 50 mM NaH₂PO₄ x H₂O, 5 mM EDTA, 0,1% BSA, 0,1% Ficoll, 0,1% PVP, 0,5% SDS, 100 µg/ml Kalbsthymus-DNA) mit einer Hybond N⁺ Membran (Amersham) durchgeführt. Plaque-Hybridisierungen wurden in 6 x SSPE (1,08 M NaCl, 60 mM NaH₂PO₄ x H₂O, 6 mM EDTA, 0,1 % BSA, 0,1% Ficoll, 0,1% PVP, 0,1% SDS, 200 µg/ml Kalbsthymus-DNA) mit einer Hybond N⁺ Membran (Amersham) durchgeführt.

### Beispiel 1

### Isolierung von YAC-Klonen aus Ls-Region der Tomate

Aus einer YAC-Bibliothek der Tomate (Martin *et al.,* 1992, Mol. Gen. Genet., 233:25-32) wurden Klone isoliert, die den Marker CD61 (Schumacher *et al.,* 1995, Mol. Gen. Genet.. 246:761-766) enthielten. Hierzu wurden zunächst DNA-Gemische, die von jeweils einer Mikrotiterplatte mit 96 YAC-Klonen stammten, mittels des üblichen PCR-Verfahrens untersucht. So konnten unter 144 solcher DNA-Gemische neun identifiziert werden, die mit den Primern CD61-F und CD61-R (Schumacher *et al.,* 1995, Mol. Gen. Genet., 246:761-766) ein PCR-Produkt- ergaben. Die Isolierung der Einzelklone erfolgte mittels Koloniehybridisierung bzw. mittels einer PCR, bei der die DNA der Klone einer Reihe bzw. Spalte einer Mikrotiter-Platte als Gemisch eingesetzt wurde. Einzelklone wurden so unter 96 Klonen einer Platte mittels 20 PCR-Reaktionen identifiziert. Es wurden insgesamt fünf YAC-Klone identifiziert, deren Insertgröße mittels Pulsfeldgelelektrophorese (Chu *et al.,* 1986, Science, 234:1582-1585) zu 280-320kb bestimmt wurde. In PCR und Southern-Experimenten wurde gezeigt, daß der YAC CD61-5 neben CD61 auch den zweiten flankierenden Marker CD65 trug und somit den Ls-Locus überspannte.

### Beispiel 2

### Isolierung von cDNA-Klonen der Ls-Region der Tomate

Für die Herstellung einer Hybridisierungssonde wurde DNA des YAC-Klons CD61-5 nach Auftrennung mittels Pulsfeldgelelektrophorese isoliert. Die Auftrennung auf diesem Pulsfeldgel erlaubte jedoch nur eine relativ grobe Präparation, so daß die eingesetzte Sonde neben dem YAC-Klon CD61-5 auch Anteile von DNA der Hefe-Chromosomen III (360kb) und VI (280kb) enthielt. Nach radioaktiver Markierung wurde diese DNA als Sonde für das Screening von 5x10⁵ pfu (plaque forming units) in einer konventionellen Plaquehybridisierung eingesetzt. Die Hybridisierung mit der YAC-Sonde lieferte eine Vielzahl von Signalen unterschiedlicher Intensität. Für das Rescreening wurden 50 Plaques verschiedener Signalstärken ausgewählt und 44 aufgereinigte Klone konnten anschließend mittels kreuzweiser Hybridisierung gruppiert werden. 23 der 44 aus dem Rescreening hervorgegangenen Klone waren nur einmal vertreten. Insgesamt wurden bei diesem Screening 29 verschiedene Transkripte identifiziert. Nach der Etablierung eines Cosmid-"Contigs" wurde die cDNA-Bibliothek nochmals mit den Cosmid-Klonen als Sonden durchmustert, um weitere cDNA-Klone zu isolieren, die beim Screening mit dem YAC61-5 als Sonde aufgrund der hohen Komplexität der Sonde nicht nachweisbar waren. In diesen Experimenten wurden drei weitere cDNA-Klone isoliert. Insgesamt wurden somit 32 verschiedene Transkripte nachgewiesen.

### Beispiel 3

### RFLP-Kartierung der isolierten cDNA-Klone der Tomate

Von den 30 identifizierten Transkripten zeigten 22 für "single"- oder "low-copy"- Sequenzen typische Hybridisierungsmuster, die eine RFLP-Kartierung erlaubten. In einer ersten RFLP-Analyse wurden die isolierten cDNA-Klone gegen Filter hybridisiert, die mit den Restriktionsenzymen EcoRI, EcoRV und Xbal verdaute DNA aus *L. esculentum, L. pennellii* sowie aus der Rückkreuzungslinie IL83 (Eshed *et al.,* 1992, Theor. Appl. Genet.; 83:1027-1034) trugen. Diese Linie, bei der das distale Ende von Chromosom 7 aus *L. pennellii* stammt, während der Rest des Genoms aus *L. esculentum*-Chromosomen besteht, erlaubt bei Vorliegen eines Polymorphismus zwischen *L. esculentum* und *L. pennellii* eine erste Grobkartierung. Stammte ein polymorphes DNA-Fragment aus der *Ls*-Region, so zeigte die Linie IL83 das *L. pennellii-*Allel, während für Fragmente aus dem Rest des Genoms das *L. esculentum-*Allel auftrat. Auf diese Weise wurden vier cDNA-Klone identifiziert, die nicht von Chromosom 7 stammen. Die Feinkartierung der 18 verbleibenden cDNA-Klone, die von Chromosom 7 stammen, erfolgte über die RFLP-Analyse der Pflanzen W23 und W24, welche Rekombinationsereignisse im Interval CD61-*Ls* bzw. *Ls*-CD65 trugen. Da Kandidaten für das Ls-Gen in dieser Analyse in Pflanze W23 sowohl das *L. esculentum-* als auch das *L. pennellii*-spezifische Fragment zeigten, während in Pflanze W24 nur das *L. esculentum-*spezifische Fragment auftrat, wurden die cDNA-Klone gegen Filter hybridisiert, die mit EcoRI, EcoRV oder XbaI gespaltene genomische DNA der beiden Elternspezies sowie der beiden Rekombinanten W23 und W24 trugen. Auf diese Weise wurden insgesamt vier cDNA-Klone identifiziert, die mit dem *Ls*-Gen kosegregierten, und somit als Kandidaten für das *Ls*-Gen in Frage kamen.

### Beispiel 4

### Erstellung und Durchmusterung einer genomischen Cosmidbank der Tomate

DNA des T-DNA/Cosmidvektors pCLD04541 (Bent *et al.,* 1994, Science, 265:1856-1860) wurde nach dem Protokoll von Sambrook *et al.,* 1989, Molecular Cloning: A Laboratory Manual. 2. Auflage, Cold Spring Harbor Laboratory Press, Cold Spring Harbor, New York, isoliert, über zwei CsCI-Gradienten gereinigt und über 3 Tage gegen TE dialysiert. Die DNA wurde mit BamHI vollständig gespalten und anschließend mit alkalischer Phosphatase dephosphoryliert, um eine Autoligation des Vektors zu verhindern. 200ng genomische Mbolpartialverdaute Tomaten-DNA und 2mg Vektor-DNA wurden mit T4-DNA-Ligase in 10ml ÜN bei 16°C ligiert. 3ml des Ligationsansatzes wurden zur Verpackung eingesetzt und in *E.coli* SURE (Stratagene) transfiziert. Der Ansatz ergab 6x10⁶ unabhängige rekombinante Bakterien. Es wurden 100 Platten mit jeweils 2500 cfu (colony forming units) plattiert und mit jeweils 10ml LB-Medium abgeschwemmt. Aus diesem Material wurde jeweils eine Glycerinkultur angelegt und eine DNA-Präparation durchgeführt. Diese 100 DNA-Pools wurden mittels einer PCR-Analyse durchmustert. Positive Pools wurden dann einer Koloniefilterhybridisierung zur Identifizierung der positiven Einzelklone unterzogen.

### Beispiel 5

### Klonierung und Sequenzierung des Ls-Gens der Tomate

Das Insert des cDNA-Klons ET, der bei der Durchmusterung der cDNA-Bank mit Cosmid G als Sonde isoliert wurde, wurde mit EcoRI ausgeschnitten und in den Vektor pGEM-11Zf(+) kloniert. Das fehlende 5'-Ende des Gens wurde mit Hilfe der RACE-Technik (Frohman *et al.,* 1988, Proc. Natl. Acad. Sci. USA 85:8998-9002) isoliert. Hierbei wurde ausgehend von einem Oligonukleotid, das spezifisch an schon bekannte Bereiche des Gens bindet, eine komplementäre DNA (cDNA) zur RNA hergestellt. Anschließend wurden mit Terminaler Transferase Desoxycytosinnukleotide an die cDNA angehängt. Mit einem zweiten genspezifischen Primer und einem Primer der an den Polydesoxycytosinschwanz bindet, wurde das 5*'*-Ende der cDNA mittels PCR amplifiziert und in den Plasmidvektor pGEM-T kloniert. Anschließend wurden die längsten dieser RACE-Klone sequenziert. Parallel zur Analyse des cDNA-Klons ET wurden Subfragmente des entsprechenden genomischen Bereichs des Cosmids G isoliert und in die Plasmidvektoren pGEM-4Z bzw. pSPORTI umkloniert. Überlappende Subfragmente wurden anschließend sequenziert. Die genomische Sequenz zeigte keine Abweichung zur Sequenz des cDNA-Klons, was bedeutet, daß das *Ls-*Gen kein Intron besitzt. Desweiteren wurden die entsprechenden Genomabschnitte der beiden Mutanten *ls*^{*1*} und *ls*^{*2*} mittels PCR mit geeigneten Primern aus den genomischen DNAs amplifiziert und in den Vektor pGEM-T kloniert. Die Sequenzanalyse dieser Produkte zeigte beim *ls*^{*1*}-Allel eine Deletion von 1,5 Kb im Vergleich zur Wildtyp-Sequenz. Neben dem Verlust der Nukleotide 1-685 des offenen Leserasters fehlen der *ls*^{*1*}-Mutante demnach auch 865 Basenpaare der 5' vom offenen Leseraster gelegenen Region, die wahrscheinlich eine regulatorische Funktion (Promotor) für die Expression besitzt. Es ist daher anzunehmen, daß die *ls*^{*1*}-Mutante kein funktionsfähiges Protein des *Ls*-Gens mehr bilden kann. Im *ls*^{*2*}-Allel wurde eine Insertion von 3 Basenpaaren sowie 3 Basenaustausche im 5'-Bereich des offenen Leserasters gefunden. Einer der Basenaustausche führt zu einem Stopcodon, das einen Abbruch der Aminosäurekette nach 24 Aminosäuren zur Folge hat. Auch hier muß von einem funktionslosen Protein ausgegangen werden. Die Vektoren pGEM-11LZf(+), pGEM-4Z, pGEM-T wurden von der Fa. Promega Corp., Madison, USA, der Vektor pSPORTI von der Fa. Life Technologies, Eggenstein, bezogen und nach Angaben der Hersteller verwendet.

### Beispiel 6

### Transformation von Pflanzen mit Ls-cDNA-Konstrukten der Tomate

Die Ls-cDNA wurde mit den genspezifischen Primem CD61-13 (5'-TTAGGGTTTTCACTCCACGC-3'; SEQ ID NO:3) und CD61-28 (5'-TCCCCTTTTTTTCCTTTCTCTC-3'; SEQ ID NO:4) mittels des üblichen PCR-Verfahrens isoliert und in den Plasmid-Vektor pGEM-4Z kloniert (GSET8). Zur Herstellung der Transformations-Konstrukte wurde die *Ls*-cDNA aus dem Plasmid GSET8 mit SalUSstI (für Sense-Konstrukt) bzw. XbaI/SstI (für Antisense-Konstrukt) herausgeschnitten und in den XhoI/SstI (Sense-Konstrukt) bzw. XbaI/SstI (Antisense-Koustrukt) geschnittenen Pflanzentransformationsvektor pBIR (Meissner, 1990, Dissertation , Universität zu Köln, Köln) ligiert. Bei den erhaltenen Klonen liegt die cDNA entweder in Sense-oder in Antisense-Orientierung zwischen dem Promotor und der Polyadenylierungsstelle des 35S-Gens des Blumenkohl-Mosaikvirus vor. Die erhaltenen Sense- bzw. Antisense-Plasmide wurden durch direkte Transformation in den *Agrobacterium tumefaciens* Stamm GV3101 (Koncz und Shell *et. al.,* 1986, Mol. Gen. Genet., 204:383-396) eingeführt. Anschließend wurden die T-DNAs der beiden unterschiedlichen Konstrukte in Blattstücke von Tomate und Tabak nach Fillatti *et. al.,* 1987, Biotech., 5:726-730 transformiert. Verschiedene transgene Pflanzen, die ein *Ls*-Antisense-Konstrukt enthalten, zeigen eine Reduktion der Seitentriebbildung.

### Beispiel 7

### Isolierung eines Ls-verwandten Gens aus Löwenmaul (Antirrhinum majus)

Mit dem cDNA-Klon ET als Sonde wurde eine genomische Phagenbibliothek aus *Antirrhinum majus* durchmustert. Die Hybridisierung erfolgte bei 55°C, d.h. unter reduzierter Stringenz. In diesem Experiment wurden 14 Klone isoliert, von denen der Klon HH13, der die stärksten Hybridisierungssignale zeigte, weiter charakterisiert wurde. Die nach Umklonierung des Phageninserts in den Plasmidvektor pGEM-HZf(+) durchgeführte Sequenzanalyse zeigte, daß das isolierte *Antirrhinum majus-Gen* hohe Sequenzähnlichkeit mit dem *Ls*-Gen aus der Tomate besitzt. Innerhalb der beiden Sequenzen konnten Bereiche identifiziert werden, in denen die abgeleitete Aminosäuresequenz vollständig konserviert ist.

### Beispiel 8

### Isolierung eines Ls-verwandten Gens aus Kartoffel (Solanum tuberosum)

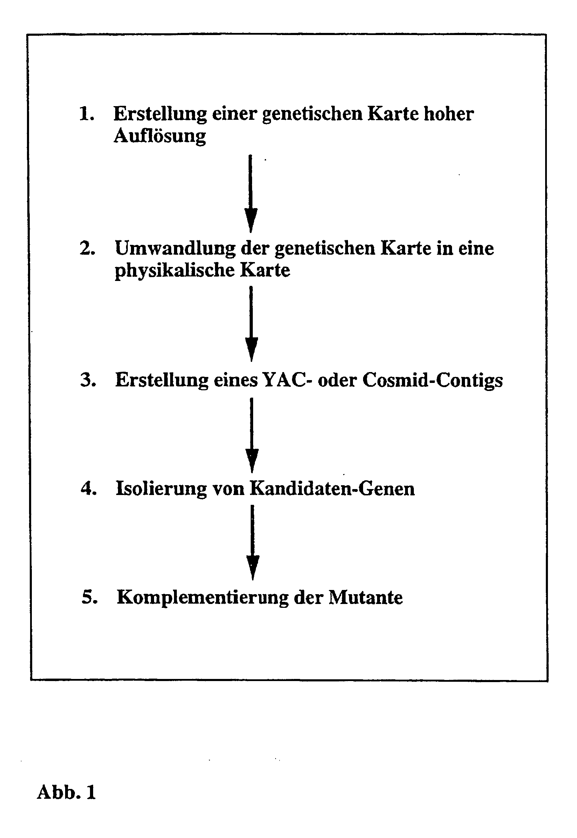

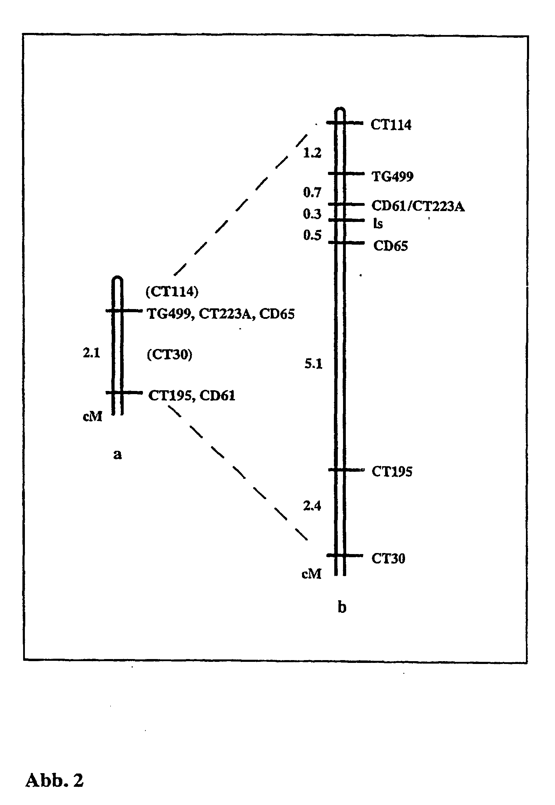

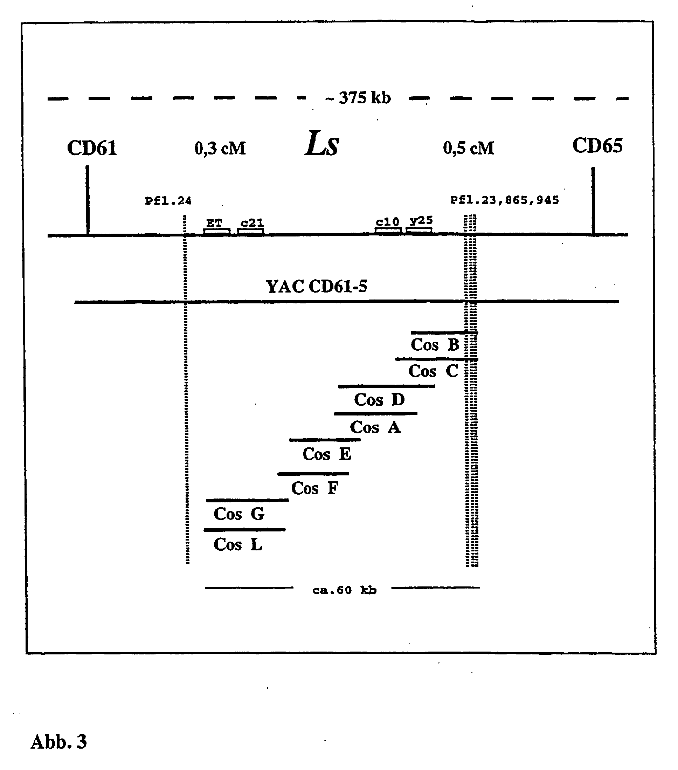

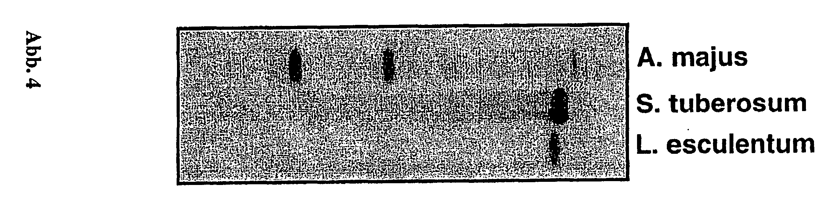

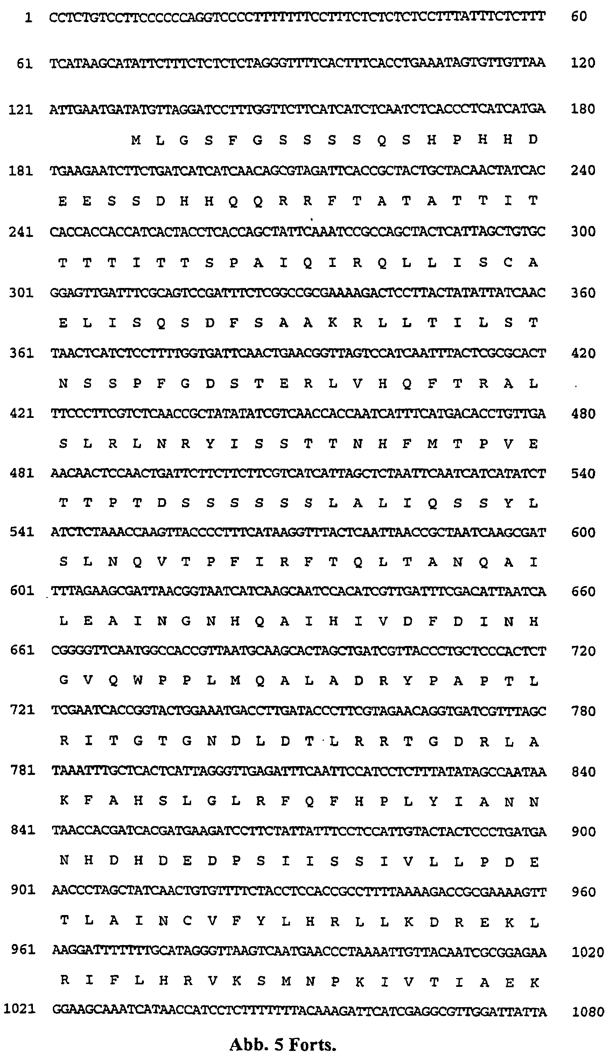

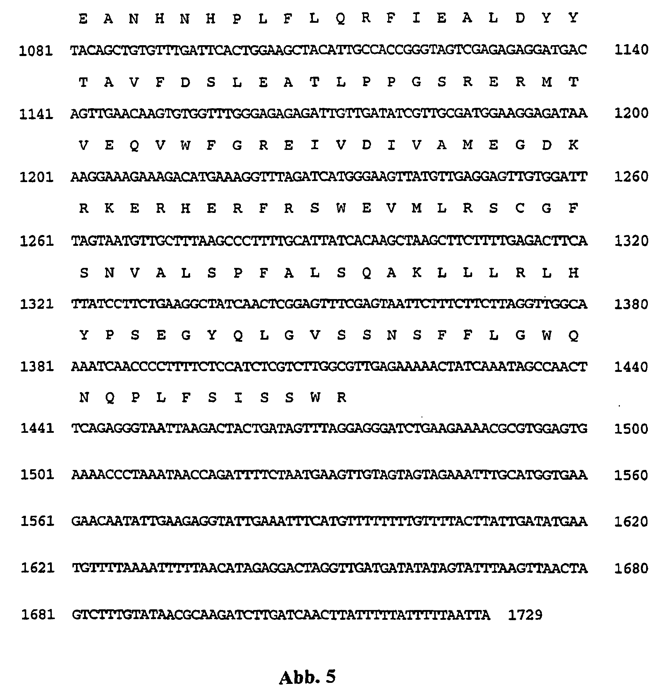

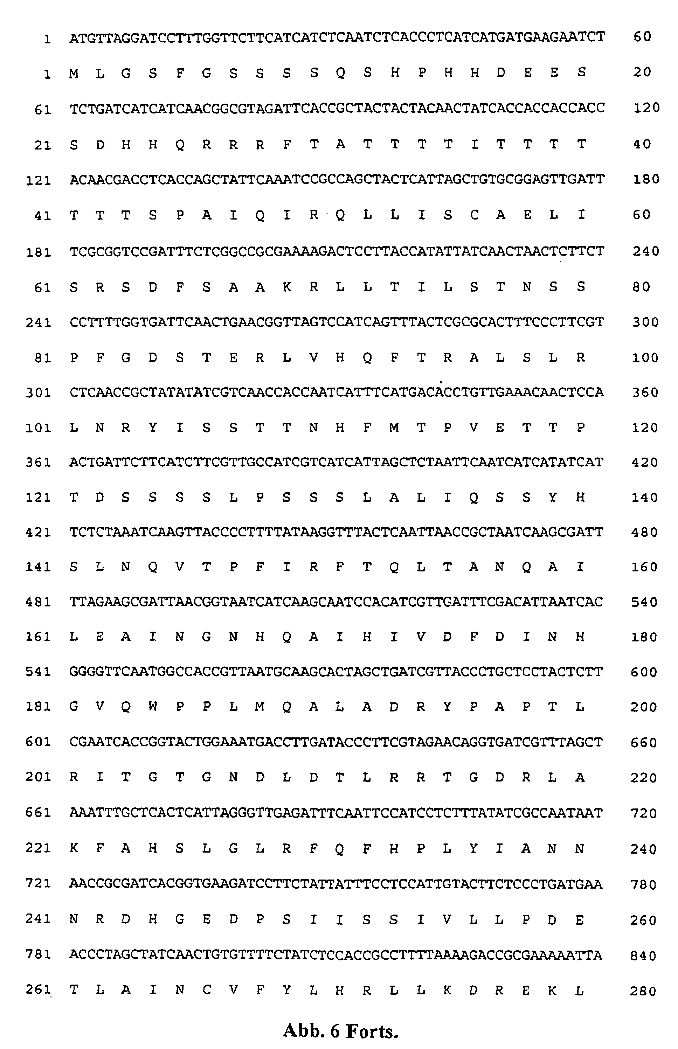

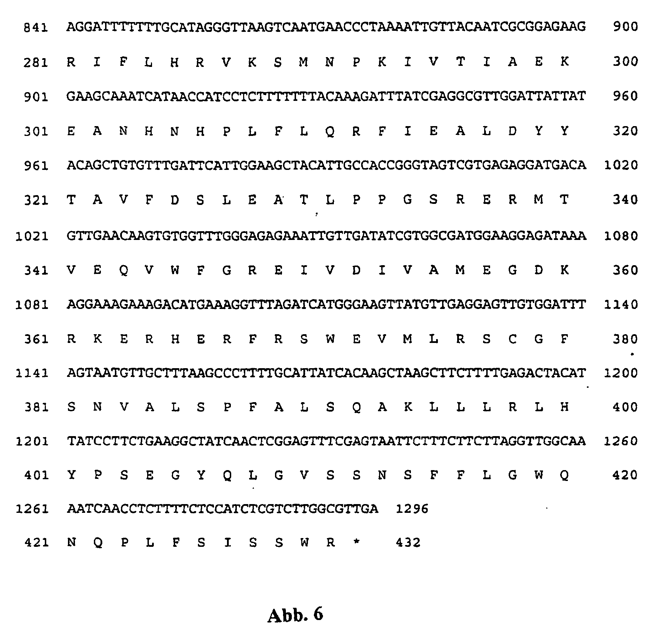

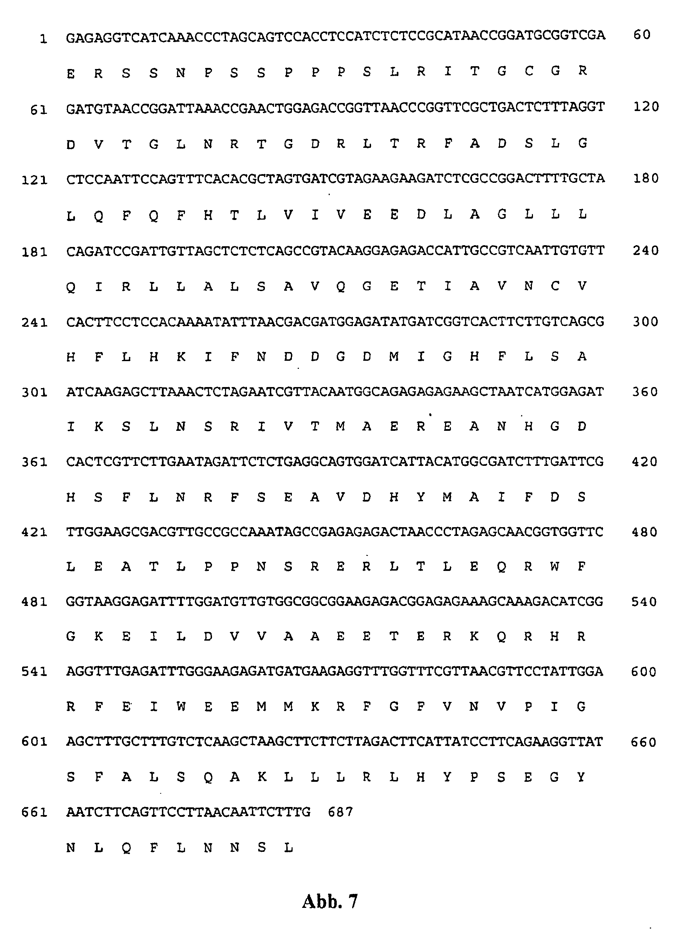

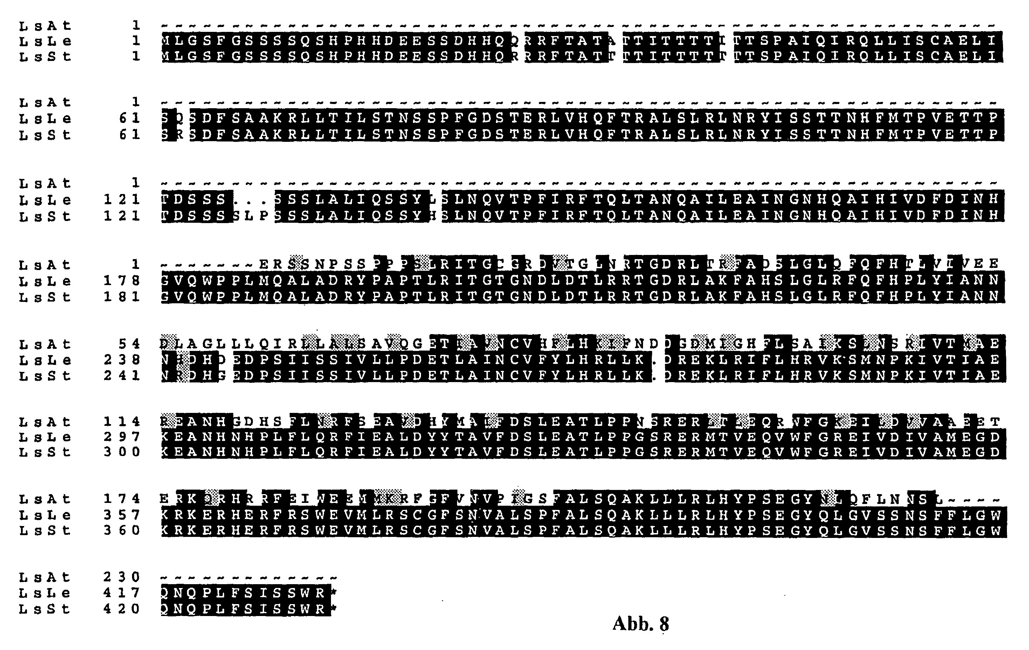

In einem Southern-Blot-Experiment unter reduzierter Stringenz bei 55°C mit der cDNA des *Ls*-Gens als Hybridisierungssonde konnte in genomischer DNA aus *Solanum tuberosum* ein DNA-Fragment nachgewiesen werden (siehe Abb. 4). Mit den genspezifischen Primem CD61-24 (5'-TTTCCCACTCAAGCCAACTC-3'; SEQ ID NO:S), CD61-6 (5'-GGTGGCAATGTAGCTTCCAG-3'; SEQ ID NO:6), PO1 (5'-TCGAGGCGTTGGATTATTATAC-3'; SEQ ID NO:7) und PO5 (5'-GGCCCCCATATCTTTTTCC-3'; SEQ ID NO:8) aus dem Ls-Gen wurden mittels des PCR-Verfahrens überlappende genomische DNA-Fragmente aus in üblicher Weise isolierter genomischer DNA von *Solanum tuberosum* isoliert. Die PCR-Reaktionen wurden wie folgt durchgeführt: Denaturierung bei 95°C für 30 Sekunden, Annealing bei 60°C für 1 Minute, Elongation bei 72°C für 2 Minuten. Dieser Cyclus wurde 30 mal wiederholt. Die erhaltenen PCR-Produkte wurden in den Plasmidvektor pGEM-T kloniert. Eine Sequenzanalyse ergab, daß die isolierten DNA-Fragmente aus *Solanum tuberosum* die Sequenzinformation für ein offenes Leseraster mit einer Kodierungskapazität von 431 Aminosäuren tragen (Abb. 6). Die DNA-Sequenz ist in SEQ ID NO:9, die von der DNA-Sequenz codierte Aminosäuresequenz ist in SEQ ID NO: 10 aufgeführt. Das Ls-homologe Gen der Kartoffel zeigt zum *Ls*-Gen der Tomate sowohl auf DNA-Ebene als auch auf Proteinebene eine Sequenzidentität von etwa 98%.

### Beispiel 9

### Isolierung eines Ls-verwandten Gens aus Arabidopsis thaliana

Zur Isolierung des *Ls*-homologen Gens aus *Arabidopsis thaliana* wurden die degenerierten Primer CD61-38 (5'-CARTGGCCNCCNYTNATGCA-3'; SEQ ID NO: 11)* und CD61-41 (5'-TGRTTYTGCCANCCNARRAA-3'; SEQ ID NO:12)* hergestellt und für PCR-Reaktionen mit in üblicher Weise isolierter genomischer DNA von *Arabidopsis thaliana* verwendet. Die PCR-Reaktionen wurden wie folgt durchgeführt: Denaturierung bei 95°C für 30 Sekunden, Annealing bei 50°C für 1 Minute, Elongation bei 72°C für 1 Minute. Dieser Cyclus wurde 35 mal wiederholt. Auf diese Weise konnte ein DNA-Fragment von etwa 700 bp amplifiziert werden, das anschließend in den Plasmid-Vektor pGEM-T kloniert wurde. Eine Sequenzanalyse zeigte, daß das isolierte DNA-Fragment aus *Arabidopsis thaliana* (SEQ ID NO:13 ) 687 bp lang war und eine hohe Sequenzähnlichkeit zum *Ls*-Gen aus *Lycopersicon esculentum* besitzt. Auf DNA-Ebene zeigt das *Arabidopsis thaliana*-DNA-Fragment zum *Ls*-Gen der Tomate eine Sequenzidentität von etwa 63 %. Auf Protein-Ebene sind etwa 55 % der Aminosäuren identisch. Die von dem isolierten DNA-Fragment (SEQ ID NO:13) codierte Aminosäuresequenz ist in SEQ ID NO: 14 aufgeführt. Unter Verwendung des isolierten DNA-Fragments kann das *Ls*-homologe Gen aus *Arabidopsis thaliana* mittels der Üblichen molekularbiologischen Standardmethoden isoliert werden.
* Bei der Beschreibung der degenerierten Primer wurde der WIPO Standard St. 23 verwendet:
R=A+G
N=A+G+C+T
Y=C+T

## Patentansprüche

1. Isolierte Desoxyribonukleinsäure mit einer Nukleotidsequenz gemäß SEQ ID NO: 1, 9 oder 13, oder eine isolierte Nukleinsäure, die mit der Nukleotidsequenz gemäß SEQ ID NO: 1, 9 oder 13 unter stringenten Bedingungen hybridisiert, wobei die isolierten Nukleinsäuren Polypeptide kodieren, die für die Steuerung der Seitentriebbildung und Petalenbildung und Abscissionszonenbildung verantwortlich sind.

2. Isoliert Desoxyribonukleinsäure mit einer Nukleotidsequenz gemäß SEQ ID NO: 1, 9 oder 13.

3. Polypeptid mit einer Aminosäuresequenz gemäß SEQ ID NO: 2, 10 oder 14.

4. Vektor, umfassend eine Nukleotidsequenz nach einem der Ansprüche 1 oder 2.

5. Mit einer exprimierbaren Desoxyribonukleinsäure gemäß Anspruch 1 transformierte Pflanzenzelle oder transformiertes Pflanzengewebe wobei die exprimierbare Desoxyribonukleinsäure gemäß Anspruch 1 stabil in das Genom der Pflanzenzelle oder das Pflanzengewebe integriert ist.

6. Pflanzenzelle oder Pflanzengewebe nach Anspruch 5, regenerierbar zu einer samenproduzierenden Pflanze.

7. Verfahren zur Herstellung von Pflanzen mit gesteuerter Seitentriebbildung und Petalenbildung und Abscissionszonenbildung, umfassend das stabile Integrieren mindestens einer Desoxyribonukleinsäure mit einer Nukleotidsequenz gemäß SEQ ID NO: 1, 9 oder 13, oder ein Fragment davon, oder einer Nukleinsäure, die mit der Nukleotidsequenz gemäß SEQ ID NO: 1, 9 oder 13 unter stringenten Bedingungen hybridisiert, wobei die Desoxyribonukleinsäure in Antisense- oder Sense-Orientierung zur für die Steuerung der Seitentriebbildung und Petalenbildung und Abscissionszonenbildung verantwortlichen endogenen Sequenz exprimiert wird, in das Genom von Pflanzenzellen oder Pflanzengeweben durch Transformation der Pflanzenzellen oder des Pflanzengewebes und Regeneration der erhaltenen Pflanzenzellen oder Pflanzengewebe zu Pflanzen.

8. Verfahren nach Anspruch 7, wobei als Pflanze eine Tomatenpflanze, eine Rapspflanze, eine Kartoffelpflanze oder eine Löwenmaulpflanze oder deren Zelle oder Gewebe verwendet wird.

9. Transformierte Pflanze, erhältlich nach Anspruch 7 oder 8.

10. Für die Nukleinsäure nach Anspruch 1 transgenes Saatgut, erhältlich von Pflanzen nach Anspruch 9.

## Claims

1. An isolated deoxyribonucleic acid having a nucleotide sequence according to SEQ ID NO: 1, 9, or 13, or an isolated nucleic acid hybridizing to a nucleotide sequence according to SEQ ID NO:1, 9, or 13 under stringent conditions, wherein the isolated nucleic acids encode polypeptides responsible for the control of the formation of side shoots and petals and abscission zones.

2. An isolated deoxyribonucleic acid having a nucleotide sequence according to SEQ ID NO:1, 9, or 13.

3. A polypeptide having an amino acid sequence according to SEQ ID NO:2, 10, or 14.

4. A vector comprising a nucleotide sequence according to any of claims 1 or 2.

5. A plant cell or plant tissue transformed with an expressible deoxyribonucleic acid according to claim 1, wherein the expressible deoxyribonucleic acid according to claim 1 is stably integrated into the genome of the plant cell or into the plant tissue.

6. The plant cell or plant tissue according to claim 5, which is regeneratable to a seed forming plant.

7. A method for generation of a plant having controlled formation of side shoots and petals and abscission zones, the method comprising the step of stably integrating at least one deoxyribonucleic acid having the nucleotide sequence according to SEQ ID NO:1, 9, or 13, or a fragment thereof, or of a nucleic acid hybridizing to the nucleic sequence according to SEQ ID NO:1, 9, or 13 under stringent conditions, wherein the deoxyribonucleic acid is expressed in antisense or sense-orientation in respect to the endogenous sequence responsible for the control of the formation of side shoots and petals and abscission zones, into the genome of plant cells or plant tissues by transformation of said plant cells or said plant tissues and regeneration of the obtained plant cells or plant tissues to plants.

8. The method according to claim 7, wherein a tomato plant, a colza plant, a potato plant, or a snapdragon plant, or cells or tissues thereof, is used as plant.

9. A transformed plant, obtainable according to claim 7 or 8.

10. A seed transgenic in the nucleic acid according to claim 1, obtainable from plants according to claim 9.

## Revendications

1. Acide désoxyribonucléotidique isolé ayant une séquence nucléotidique conformément à SEQ ID NO : 1, 9 ou 13, ou acide nucléique isolé, qui hybride avec la séquence nucléotidique conformément à SEQ ID NO : 1, 9 ou 13 en conditions stringentes, les acides nucléiques codant un polypeptide qui est responsable de la régulation de la formation des pousses latérales et de la formation des pétales et de la formation des zones d'abscission.

2. Acide désoxyribonucléotidique isolé ayant une séquence nucléotidique conformément à SEQ ID NO : 1, 9 ou 13.

3. Polypeptide ayant une séquence d'acides aminés conformément à SEQ ID NO : 2, 10 ou 14.

4. Vecteur comprenant une séquence nucléotidique selon l'une des revendications 1 ou 2.

5. Cellule végétale ou tissu végétal transformé avec un acide désoxyribonucléotidique isolé exprimable selon la revendication 1, l'acide désoxyribonucléotidique isolé exprimable selon la revendication 1 étant intégré de manière stable dans le génome de la cellule végétale ou du tissu végétal.

6. Cellule végétale ou tissu végétal selon la revendication 5, pouvant être régénéré(e) en une plante productrice de semence.

7. Procédé pour la fabrication de plantes ayant une régulation de la formation des pousses latérales et de la formation de pétales et de la formation des zones d'abscission, comprenant l'intégration stable d'au moins un acide désoxyribonucléotidique isolé ayant une séquence nucléotidique conformément à SEQ ID NO : 1, 9 ou 13 ou un fragment de celle-ci, ou un acide nucléique qui hybride avec la séquence nucléotidique conformément à SEQ ID NO : 1, 9 ou 13 en conditions stringentes, l'acide désoxyribonucléotidique isolé exprimant dans l'orientation anti-sens ou sens une séquence endogène responsable de la régulation de la formation des pousses latérales et de la formation de pétales et de la formation des zones d'abscission, dans le génome de cellules végétales ou tissus végétaux par transformation des cellules végétales ou du tissu végétal et régénération des cellules végétales ou tissus végétaux obtenu(e)s en plantes.

8. Procédé selon la revendication 7, dans lequel on utilise en tant que plante une plante de tomate, une plante de colza, une plante de pomme de terre, une plante de muflier ou leurs cellules ou tissus

9. Plante transformée, pouvant être obtenue selon la revendication 7 ou 8.

10. Semences transgéniques pour l'acide nucléique selon la revendication 1 pouvant être obtenues à partir de plantes selon la revendication 9.


### SEQUENZPROTOKOLL

(1) ALLGEMEINE ANGABEN:
   (i) ANMELDER:
      (A) NAME: Nikolaus (Klaus) Theres
      (B) STRASSE: Schiffgesweg 30
      (C) ORT: Pulheim
      (D) BUNDESLAND: NRW
      (E) LAND: Deutschland
      (F) POSTLEITZAHL: 50259
      (G) TELEFON: 02234-89386
   (ii) BEZEICHNUNG DER ERFINDUNG: Pflanzen mit gesteuerter
      Seitentriebbildung und/oder gesteuerter Anscissionszonenbildung
   (iii) ANZAHL DER SEQUENZEN: 14
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: Patent In Release #1.0, Version #1.30 (EPA)
(2) ANGABEN ZU SEQ ID NO: 1:
   (i) SEQUENZKENNZEICHEN:
      (A) LÄNGE: 1729 Basenpaare
      (B) ART: Nucleotid
      (C) STRANGFORM: Doppelstrang
      (D) TOPOLOGIE: linear
   (ii) ART DES MOLEKÜLS: cDNA
   (iii) HYPOTHETISCH: NEIN
   (vi) URSPRÜNLICHE HERKUNFT:
      (A) ORGANISMUS: Lycopersicon esculentum
   (xi) SEQUENZBESCHREIBUNG: SEQ ID NO: 1:
   CCTCTGTCCT TCCCCCCAGG TCCCCTTTTT TTCCTTTCTC TCTCTCCTTT ATTTCTCTTT 60
   TCATAAGCAT ATTCTTTCTC TCTCTAGGGT TTTCACTTTC ACCTGAAATA GTGTTGTTAA 120
   ATTGAATGAT ATGTTAGGAT CCTTTGGTTC TTCATCATCT CAATCTCACC CTCATCATGA 180
   TGAAGAATCT TCTGATCATC ATCAACAGCG TAGATTCACC GCTACTGCTA CAACTATCAC 240
   CACCACCACC ATCACTACCT CACCAGCTAT TCAAATCCGC CAGCTACTCA TTAGCTGTGC 300
   GGAGTTGATT TCGCAGTCCG ATTTCTCGGC CGCGAAAAGA CTCCTTACTA TATTATCAAC 360
   TAACTCATCT CCTTTTGGTG ATTCAACTGA ACGGTTAGTC CATCAATTTA CTCGCGCACT 420
   TTCCCTTCGT CTCAACCGCT ATATATCGTC AACCACCAAT CATTTCATGA CACCTGTTGA 480
   AACAACTCCA ACTGATTCTT CTTCTTCGTC ATCATTAGCT CTAATTCAAT CATCATATCT 540
   ATCTCTAAAC CAAGTTACCC CTTTCATAAG GTTTACTCAA TTAACCGCTA ATCAAGCGAT    600
   TTTAGAAGCG ATTAACGGTA ATCATCAAGC AATCCACATC GTTGATTTCG ACATTAATCA    660
   CGGGGTTCAA TGGCCACCGT TAATGCAAGC ACTAGCTGAT CGTTACCCTG CTCCCACTCT    720
   TCGAATCACC GGTACTGGAA ATGACCTTGA TACCCTTCGT AGAACAGGTG ATCGTTTAGC    780
   TAAATTTGCT CACTCATTAG GGTTGAGATT TCAATTCCAT CCTCTTTATA TAGCCAATAA    840
   TAACCACGAT CACGATGAAG ATCCTTCTAT TATTTCCTCC ATTGTACTAC TCCCTGATGA    900
   AACCCTAGCT ATCAACTGTG TTTTCTACCT CCACCGCCTT TTAAAAGACC GCGAAAAGTT    960
   AAGGATTTTT TTGCATAGGG TTAAGTCAAT GAACCCTAAA ATTGTTACAA TCGCGGAGAA   1020
   GGAAGCAAAT CATAACCATC CTCTTTTTTT ACAAAGATTC ATCGAGGCGT TGGATTATTA   1080
   TACAGCTGTG TTTGATTCAC TGGAAGCTAC ATTGCCACCG GGTAGTCGAG AGAGGATGAC   1140
   AGTTGAACAA GTGTGGTTTG GGAGAGAGAT TGTTGATATC GTTGCGATGG AAGGAGATAA   1200
   AAGGAAAGAA AGACATGAAA GGTTTAGATC ATGGGAAGTT ATGTTGAGGA GTTGTGGATT   1260
   TAGTAATGTT GCTTTAAGCC CTTTTGCATT ATCACAAGCT AAGCTTCTTT TGAGACTTCA   1320
   TTATCCTTCT GAAGGCTATC AACTCGGAGT TTCGAGTAAT TCTTTCTTCT TAGGTTGGCA   1380
   AAATCAACCC CTTTTCTCCA TCTCGTCTTG GCGTTGAGAA AAACTATCAA ATAGCCAACT   1440
   TCAGAGGGTA ATTAAGACTA CTGATAGTTT AGGAGGGATC TGAAGAAAAC GCGTGGAGTG   1500
   AAAACCCTAA ATAACCAGAT TTTCTAATGA AGTTGTAGTA GTAGAAATTT GCATGGTGAA   1560
   GAACAATATT GAAGAGGTAT TGAAATTTCA TGTTTTTTTT GTTTTACTTA TTGATATGAA   1620
   TGTTTTAAAA TTTTTAACAT AGAGGACTAG GTTGATGATA TATAGTATTT AAGTTAACTA   1680
   GTCTTTGTAT AACGCAAGAT CTTGATCAAC TTATTTTTAT TTTTAATTA               1729
(2) ANGABEN ZU SEQ ID NO: 2:
   (i) SEQUENZKENNZEICHEN:
      (A) LÄNGE: 428 Aminosäuren
      (B) ART: Aminosäure
      (C) STRANGFORM: Einzelstrang
      (D) TOPOLOGIE: linear
   (ii) ART DES MOLEKÜLS: Protein
   vi) URSPRÜNLICHE HERKUNFT:
      (A) ORGANISMUS: Lycopersicon esculentum
   (xi) SEQUENZBESCHREIBUNG: SEQ ID NO: 2:
   Met Leu Gly Ser Phe Gly Ser Ser Ser Ser Gln Ser His Pro His His
   1               5                   10                  15
   Asp Glu Glu Ser Ser Asp His His Gln Gln Arg Arg Phe Thr Ala Thr
   20 25 30
   Ala Thr Thr Ile Thr Thr Thr Thr Ile Thr Thr Ser Pro Ala Ile Gln
   35 40 45
   Ile Arg Gln Leu Leu Ile Ser Cys Ala Glu Leu Ile Ser Gln Ser Asp
   50 55 60
   Phe Ser Ala Ala Lys Arg Leu Leu Thr Ile Leu Ser Thr Asn Ser Ser
   65 70 75 80
   Pro Phe Gly Asp Ser Thr Glu Arg Leu Val His Gln Phe Thr Arg Ala
   85 90 95
   Leu Ser Leu Arg Leu Asn Arg Tyr Ile Ser Ser Thr Thr Asn His Phe
   100 105 110
   Met Thr Pro Val Glu Thr Thr Pro Thr Asp Ser Ser Ser Ser Ser Ser
   115 120 125
   Leu Ala Leu Ile Gln Ser Ser Tyr Leu Ser Leu Asn Gln Val Thr Pro
   130 135 140
   Phe Ile Arg Phe Thr Gln Leu Thr Ala Asn Gln Ala Ile Leu Glu Ala
   145 150 155 160
   Ile Asn Gly Asn His Gln Ala Ile His Ile Val Asp Phe Asp Ile Asn
   165 170 175
   His Gly Val Gln Trp Pro Pro Leu Met Gln Ala Leu Ala Asp Arg Tyr
   180 185 190
   Pro Ala Pro Thr Leu Arg Ile Thr Gly Thr Gly Asn Asp Leu Asp Thr
   195 200 205
   Leu Arg Arg Thr Gly Asp Arg Leu Ala Lys Phe Ala His Ser Leu Gly
   210 215 220
   Leu Arg Phe Gln Phe His Pro Leu Tyr Ile Ala Asn Asn Asn His Asp
   225 230 235 240
   His Asp Glu Asp Pro Ser Ile Ile Ser Ser Ile Val Leu Leu Pro Asp
   245 250 255
   Glu Thr Leu Ala Ile Asn Cys Val Phe Tyr Leu His Arg Leu Leu Lys
   260 265 270
   Asp Arg Glu Lys Leu Arg Ile Phe Leu His Arg Val Lys Ser Met Asn
   275 280 285
   Pro Lys Ile Val Thr Ile Ala Glu Lys Glu Ala Asn His Asn His Pro
   290 295 300
   Leu Phe Leu Gln Arg Phe Ile Glu Ala Leu Asp Tyr Tyr Thr Ala Val
   305 310 315 320
   Phe Asp Ser Leu Glu Ala Thr Leu Pro Pro Gly Ser Arg Glu Arg Met
   325 330 335
   Thr Val Glu Gln Val Trp Phe Gly Arg Glu Ile Val Asp Ile Val Ala
               340                 345                 350
   Met Glu Gly Asp Lys Arg Lys Glu Arg His Glu Arg Phe Arg Ser Trp
           355                 360                 365
   Glu Val Met Leu Arg Ser Cys Gly Phe Ser Asn Val Ala Leu Ser Pro
       370                 375                 380
   Phe Ala Leu Ser Gln Ala Lys Leu Leu Leu Arg Leu His Tyr Pro Ser
   385                 390                 395                 400
   Glu Gly Tyr Gln Leu Gly Val Ser Ser Asn Ser Phe Phe Leu Gly Trp
                   405                 410                 415
   Gln Asn Gln Pro Leu Phe Ser Ile Ser Ser Trp Arg
               420                 425
(2) ANGABEN ZU SEQ ID NO: 3:
   (i) SEQUENZKENNZEICHEN:
      (A) LÄNGE: 20 Basenpaare
      (B) ART: Nucleotid
      (C) STRANGFORM:Einzelstrang
      (D) TOPOLOGIE: linear
   (ii) ART DES MOLEKÜLS: synthetische DNA
   (iii) HYPOTHETISCH: NEIN
   (xi) SEQUENZBESCHREIBUNG: SEQ ID NO: 3:
   TTAGGGTTTT CACTCCACGC                                          20
(2) ANGABEN ZU SEQ ID NO: 4:
   (i) SEQUENZKENNZEICHEN:
      (A) LÄNGE: 22 Basenpaare
      (B) ART: Nucleotid
      (C) STRANGFORM:Einzelstrang
      (D) TOPOLOGIE: linear
   (ii) ART DES MOLEKÜLS: synthetische DNA
   (iii) HYPOTHETISCH: NEIN
   (xi) SEQUENZBESCHREIBUNG: SEQ ID NO: 4:
   TCCCCTTTTT TTCCTTTCTC TC                                       22
(2) ANGABEN ZU SEQ ID NO: 5:
   (i) SEQUENZKENNZEICHEN:
      (A) LÄNGE: 20 Basenpaare
      (B) ART: Nucleotid
      (C) STRANGFORM:Einzelstrang
      (D) TOPOLOGIE: linear
   (ii) ART DES MOLEKÜLS: synthetische DNA
   (iii) HYPOTHETISCH: NEIN
   (xi) SEQUENZBESCHREIBUNG: SEQ ID NO: 5:
   TTTCCCACTC AAGCCAACTC 20
(2) ANGABEN ZU SEQ ID NO: 6:
   (i) SEQUENZKENNZEICHEN:
      (A) LÄNGE: 20 Basenpaare
      (B) ART: Nucleotid
      (C) STRANGFORM:Einzelstrang
      (D) TOPOLOGIE: linear
   (ii) ART DES MOLEKÜLS: synthetische DNA
   (iii) HYPOTHETISCH: NEIN
   (xi) SEQUENZBESCHREIBUNG: SEQ ID NO: 6:
   GGTGGCAATG TAGCTTCCAG 20
(2) ANGABEN ZU SEQ ID NO: 7:
   (i) SEQUENZKENNZEICHEN:
      (A) LÄNGE: 22 Basenpaare
      (B) ART: Nucleotid
      (C) STRANGFORM:Einzelstrang
      (D) TOPOLOGIE: linear
   (ii) ART DES MOLEKÜLS: synthetische DNA
   (iii) HYPOTHETISCH: NEIN
   (xi) SEQUENZBESCHREIBUNG: SEQ ID NO: 7:
   TCGAGGCGTT GGATTATTAT AC 22
(2) ANGABEN ZU SEQ ID NO: 8:
   (i) SEQUENZKENNZEICHEN:
      (A) LÄNGE: 19 Basenpaare
      (B) ART: Nucleotid
      (C) STRANGFORM:Einzelstrang
      (D) TOPOLOGIE: linear
   (ii) ART DES MOLEKÜLS: synthetische DNA
   (iii) HYPOTHETISCH: NEIN
   (xi) SEQUENZBESCHREIBUNG: SEQ ID NO: 8:
   GGCCCCCATA TCTTTTTCC                                                  19
(2) ANGABEN ZU SEQ ID NO: 9:
   (i) SEQUENZKENNZEICHEN:
      (A) LÄNGE: 1296 Basenpaare
      (B) ART: Nucleotid
      (C) STRANGFORM: Doppelstrang
      (D) TOPOLOGIE: linear
   (ii) ART DES MOLEKÜLS: cDNA
   (iii) HYPOTHETISCH: NEIN
   (vi) URSPRÜNLICHE HERKUNFT:
      (A) ORGANISMUS: Solanum tuberosum
   (xi) SEQUENZBESCHREIBUNG: SEQ ID NO: 9:
   ATGTTAGGAT CCTTTGGTTC TTCATCATCT CAATCTCACC CTCATCATGA TGAAGAATCT      60
   TCTGATCATC ATCAACGGCG TAGATTCACC GCTACTACTA CAACTATCAC CACCACCACC     120
   ACAACGACCT CACCAGCTAT TCAAATCCGC CAGCTACTCA TTAGCTGTGC GGAGTTGATT     180
   TCGCGGTCCG ATTTCTCGGC CGCGAAAAGA CTCCTTACCA TATTATCAAC TAACTCTTCT     240
   CCTTTTGGTG ATTCAACTGA ACGGTTAGTC CATCAGTTTA CTCGCGCACT TTCCCTTCGT     300
   CTCAACCGCT ATATATCGTC AACCACCAAT CATTTCATGA CACCTGTTGA AACAACTCCA     360
   ACTGATTCTT CATCTTCGTT GCCATCGTCA TCATTAGCTC TAATTCAATC ATCATATCAT     420
   TCTCTAAATC AAGTTACCCC TTTTATAAGG TTTACTCAAT TAACCGCTAA TCAAGCGATT     480
   TTAGAAGCGA TTAACGGTAA TCATCAAGCA ATCCACATCG TTGATTTCGA CATTAATCAC     540
   GGGGTTCAAT GGCCACCGTT AATGCAAGCA CTAGCTGATC GTTACCCTGC TCCTACTCTT     600
   CGAATCACCG GTACTGGAAA TGACCTTGAT ACCCTTCGTA GAACAGGTGA TCGTTTAGCT     660
   AAATTTGCTC ACTCATTAGG GTTGAGATTT CAATTCCATC CTCTTTATAT CGCCAATAAT     720
   AACCGCGATC ACGGTGAAGA TCCTTCTATT ATTTCCTCCA TTGTACTTCT CCCTGATGAA     780
   ACCCTAGCTA TCAACTGTGT TTTCTATCTC CACCGCCTTT TAAAAGACCG CGAAAAATTA     840
   AGGATTTTTT TGCATAGGGT TAAGTCAATG AACCCTAAAA TTGTTACAAT CGCGGAGAAG     900
   GAAGCAAATC ATAACCATCC TCTTTTTTTA CAAAGATTTA TCGAGGCGTT GGATTATTAT     960
   ACAGCTGTGT TTGATTCATT GGAAGCTACA TTGCCACCGG GTAGTCGTGA GAGGATGACA    1020
   GTTGAACAAG TGTGGTTTGG GAGAGAAATT GTTGATATCG TGGCGATGGA AGGAGATAAA    1080
   AGGAAAGAAA GACATGAAAG GTTTAGATCA TGGGAAGTTA TGTTGAGGAG TTGTGGATTT    1140
   AGTAATGTTG CTTTAAGCCC TTTTGCATTA TCACAAGCTA AGCTTCTTTT GAGACTACAT    1200
   TATCCTTCTG AAGGCTATCA ACTCGGAGTT TCGAGTAATT CTTTCTTCTT AGGTTGGCAA    1260
   AATCAACCTC TTTTCTCCAT CTCGTCTTGG CGTTGA                              1296
(2) ANGABEN ZU SEQ ID NO: 10:
   (i) SEQUENZKENNZEICHEN:
      (A) LÄNGE: 431 Aminosäuren
      (B) ART: Aminosäure
      (C) STRANGFORM: Einzelstrang
      (D) TOPOLOGIE: linear
   (ii) ART DES MOLEKÜLS: Protein
   (vi) URSPRÜNLICHE HERKUNFT:
      (A) ORGANISMUS: Solanum tuberosum
   (xi) SEQUENZBESCHREIBUNG: SEQ ID NO: 10:
   Met Leu Gly Ser Phe Gly Ser Ser Ser Ser Gln Ser His Pro His His
   1               5                   10                  15
   Asp Glu Glu Ser Ser Asp His His Gln Arg Arg Arg Phe Thr Ala Thr
               20                  25                  30
   Thr Thr Thr Ile Thr Thr Thr Thr Thr Thr Thr Ser Pro Ala Ile Gln
           35                  40                  45
   Ile Arg Gln Leu Leu Ile Ser Cys Ala Glu Leu Ile Ser Arg Ser Asp
       50                  55                  60
   Phe Ser Ala Ala Lys Arg Leu Leu Thr Ile Leu Ser Thr Asn Ser Ser
   65                  70                  75                  80
   Pro Phe Gly Asp Ser Thr Glu Arg Leu Val His Gln Phe Thr Arg Ala
                   85                  90                  95
   Leu Ser Leu Arg Leu Asn Arg Tyr Ile Ser Ser Thr Thr Asn His Phe
               100                 105                 110
   Met Thr Pro Val Glu Thr Thr Pro Thr Asp Ser Ser Ser Ser Leu Pro
           115                 120                 125
   Ser Ser Ser Leu Ala Leu Ile Gln Ser Ser Tyr His Ser Leu Asn Gln
       130                 135                 140
   Val Thr Pro Phe Ile Arg Phe Thr Gln Leu Thr Ala Asn Gln Ala Ile
   145                 150                 155                 160
   Leu Glu Ala Ile Asn Gly Asn His Gln Ala Ile His Ile Val Asp Phe
                   165                 170                 175
   Asp Ile Asn His Gly Val Gln Trp Pro Pro Leu Met Gln Ala Leu Ala
               180                 185                 190
   Asp Arg Tyr Pro Ala Pro Thr Leu Arg Ile Thr Gly Thr Gly Asn Asp
           195                 200                 205
   Leu Asp Thr Leu Arg Arg Thr Gly Asp Arg Leu Ala Lys Phe Ala His
       210                 215                 220
   Ser Leu Gly Leu Arg Phe Gln Phe His Pro Leu Tyr Ile Ala Asn Asn
   225                 230                 235                 240
   Asn Arg Asp His Gly Glu Asp Pro Ser Ile Ile Ser Ser Ile Val Leu
                   245                 250                 255
   Leu Pro Asp Glu Thr Leu Ala Ile Asn Cys Val Phe Tyr Leu His Arg
               260                 265                 270
   Leu Leu Lys Asp Arg Glu Lys Leu Arg Ile Phe Leu His Arg Val Lys
           275                 280                 285
   Ser Met Asn Pro Lys Ile Val Thr Ile Ala Glu Lys Glu Ala Asn His
       290                 295                 300
   Asn His Pro Leu Phe Leu Gln Arg Phe Ile Glu Ala Leu Asp Tyr Tyr
   305                 310                 315                 320
   Thr Ala Val Phe Asp Ser Leu Glu Ala Thr Leu Pro Pro Gly Ser Arg
                   325                 330                 335
   Glu Arg Met Thr Val Glu Gln Val Trp Phe Gly Arg Glu Ile Val Asp
               340                 345                 350
   Ile Val Ala Met Glu Gly Asp Lys Arg Lys Glu Arg His Glu Arg Phe
           355                 360                 365
   Arg Ser Trp Glu Val Met Leu Arg Ser Cys Gly Phe Ser Asn Val Ala
       370                 375                 380
   Leu Ser Pro Phe Ala Leu Ser Gln Ala Lys Leu Leu Leu Arg Leu His
   385                 390                 395                 400
   Tyr Pro Ser Glu Gly Tyr Gln Leu Gly Val Ser Ser Asn Ser Phe Phe
                   405                 410                 415
   Leu Gly Trp Gln Asn Gln Pro Leu Phe Ser Ile Ser Ser Trp Arg
               420                 425                 430
(2) ANGABEN ZU SEQ ID NO: 11:
   (i) SEQUENZKENNZEICHEN:
      (A) LÄNGE: 20 Basenpaare
      (B) ART: Nucleotid
      (C) STRANGFORM:Einzelstrang
      (D) TOPOLOGIE: linear
   (ii) ART DES MOLEKÜLS: synthetische DNA
   (iii) HYPOTHETISCH: NEIN
   (xi) SEQUENZBESCHREIBUNG: SEQ ID NO: 11:
   CARTGGCCNC CNYTNATGCA                                              20
(2) ANGABEN ZU SEQ ID NO: 12:
   (i) SEQUENZKENNZEICHEN:
      (A) LÄNGE: 20 Basenpaare
      (B) ART: Nucleotid
      (C) STRANGFORM:Einzelstrang
      (D) TOPOLOGIE: linear
   (ii) ART DES MOLEKÜLS: synthetische DNA
   (iii) HYPOTHETISCH: NEIN
   (xi) SEQUENZBESCHREIBUNG: SEQ ID NO: 12:
   TGRTTYTGCC ANCCNARRAA 20
(2) ANGABEN ZU SEQ ID NO: 13:
   (i) SEQUENZKENNZEICHEN:
      (A) LÄNGE: 687 Basenpaare
      (B) ART: Nucleotid
      (C) STRANGFORM: Doppelstrang
      (D) TOPOLOGIE: linear
   (ii) ART DES MOLEKÜLS: cDNA
   (iii) HYPOTHETISCH: NEIN
   (vi) URSPRÜNLICHE HERKUNFT:
      (A) ORGANISMUS: Arabidopsis thaliana
   (xi) SEQUENZBESCHREIBUNG: SEQ ID NO: 13:
   GAGAGGTCAT CAAACCCTAG CAGTCCACCT CCATCTCTCC GCATAACCGG ATGCGGTCGA    60
   GATGTAACCG GATTAAACCG AACTGGAGAC CGGTTAACCC GGTTCGCTGA CTCTTTAGGT   120
   CTCCAATTCC AGTTTCACAC GCTAGTGATC GTAGAAGAAG ATCTCGCCGG ACTTTTGCTA   180
   CAGATCCGAT TGTTAGCTCT CTCAGCCGTA CAAGGAGAGA CCATTGCCGT CAATTGTGTT   240
   CACTTCCTCC ACAAAATATT TAACGACGAT GGAGATATGA TCGGTCACTT CTTGTCAGCG   300
   ATCAAGAGCT TAAACTCTAG AATCGTTACA ATGGCAGAGA GAGAAGCTAA TCATGGAGAT   360
   CACTCGTTCT TGAATAGATT CTCTGAGGCA GTGGATCATT ACATGGCGAT CTTTGATTCG   420
   TTGGAAGCGA CGTTGCCGCC AAATAGCCGA GAGAGACTAA CCCTAGAGCA ACGGTGGTTC   480
   GGTAAGGAGA TTTTGGATGT TGTGGCGGCG GAAGAGACGG AGAGAAAGCA AAGACATCGG   540
   AGGTTTGAGA TTTGGGAAGA GATGATGAAG AGGTTTGGTT TCGTTAACGT TCCTATTGGA   600
   AGCTTTGCTT TGTCTCAAGC TAAGCTTCTT CTTAGACTTC ATTATCCTTC AGAAGGTTAT   660
   AATCTTCAGT TCCTTAACAA TTCTTTG                                        687
(2) ANGABEN ZU SEQ ID NO: 14:
   (i) SEQUENZKENNZEICHEN:
      (A) LÄNGE: 229 Aminosäuren
      (B) ART: Aminosäure
      (C) STRANGFORM: Einzelstrang
      (D) TOPOLOGIE: linear
   (ii) ART DES MOLEKÜLS: Protein
   (vi) URSPRÜNLICHE HERKUNFT:
      (A) ORGANISMUS: Arabidopsis thaliana
   (xi) SEQUENZBESCHREIBUNG: SEQ ID NO: 14:
   Glu Arg Ser Ser Asn Pro Ser Ser Pro Pro Pro Ser Leu Arg Ile Thr
   1               5                   10                  15
   Gly Cys Gly Arg Asp Val Thr Gly Leu Asn Arg Thr Gly Asp Arg Leu
               20                  25                  30
   Thr Arg Phe Ala Asp Ser Leu Gly Leu Gln Phe Gln Phe His Thr Leu
           35                  40                  45
   Val Ile Val Glu Glu Asp Leu Ala Gly Leu Leu Leu Gln Ile Arg Leu
       50                  55                  60
   Leu Ala Leu Ser Ala Val Gln Gly Glu Thr Ile Ala Val Asn Cys Val
   65                  70                  75                  80
   His Phe Leu His Lys Ile Phe Asn Asp Asp Gly Asp Met Ile Gly His
                   85                  90                  95
   Phe Leu Ser Ala Ile Lys Ser Leu Asn Ser Arg Ile Val Thr Met Ala
               100                 105                 110
   Glu Arg Glu Ala Asn His Gly Asp His Ser Phe Leu Asn Arg Phe Ser
           115                 120                 125
   Glu Ala Val Asp His Tyr Met Ala Ile Phe Asp Ser Leu Glu Ala Thr
       130                 135                 140
   Leu Pro Pro Asn Ser Arg Glu Arg Leu Thr Leu Glu Gln Arg Trp Phe
   145                 150                 155                 160
   Gly Lys Glu Ile Leu Asp Val Val Ala Ala Glu Glu Thr Glu Arg Lys
                   165                 170                 175
   Gln Arg His Arg Arg Phe Glu Ile Trp Glu Glu Met Met Lys Arg Phe
               180                 185                 190
   Gly Phe Val Asn Val Pro Ile Gly Ser Phe Ala Leu Ser Gln Ala Lys
           195                 200                 205
   Leu Leu Leu Arg Leu His Tyr Pro Ser Glu Gly Tyr Asn Leu Gln Phe
       210                 215                 220
   Leu Asn Asn Ser Leu
   225